(19) Europäisches Patentamt
European Patent Office
Office européen des brevets

(11) Publication number : 0 434 094 B1

(12) # EUROPEAN PATENT SPECIFICATION

(45) Date of publication of patent specification : 31.03.93 Bulletin 93/13

(51) Int. Cl.$^5$ : C07C 255/43, A61K 31/275

(21) Application number : 90125244.5

(22) Date of filing : 21.12.90

(54) **Methods of using 2-isopropyl-2-phenylaminovaleronitriles immunosuppresants and vasodilators.**

(30) Priority : 21.12.89 US 456007

(43) Date of publication of application : 26.06.91 Bulletin 91/26

(45) Publication of the grant of the patent : 31.03.93 Bulletin 93/13

(84) Designated Contracting States : AT BE CH DE DK ES FR GB GR IT LI LU NL SE

(56) References cited : EP-A- 0 271 013

(73) Proprietor : G.D. Searle & Co.
P.O. Box 5110
Chicago Illinois 60680 (US)

(72) Inventor : Chi-Dean Liang
1416 Evergreen Terrace
Glenview Illinois 60025 (US)
Inventor : Mueller, Richard August
562 Stonegate Terrace
Glencoe, Ill. 60022 (US)
Inventor : Mc Kearn, John P.
18827 Highwood Estates Drive
Pacific, Missouri 63069 (US)
Inventor : Strom, Terry B.
22 Kennard Rd.
Brookline, Massachusetts 02146 (US)
Inventor : Wieder, Kenneth J.
47 Bingham Ave.
Dedham, Massachusetts 02026 (US)

(74) Representative : Beil, Hans Chr., Dr. et al
Beil, Wolff und Beil, Rechtsanwälte
Adelonstrasse 58 Postfach 80 01 40
W-6230 Frankfurt am Main 80 (DE)

EP 0 434 094 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention provides novel methods of using novel compounds and compositions, such compounds being pharmacologically useful as antihypertensives and for immunosuppression of a mammal, and being especially useful for immunosuppression in a mammal in conjunction with administration of other immunosuppressing drugs, especially cyclosporin (also known as cyclosporin A). More specifically, the method of the present invention is to cause immunosuppression by administration of a class of 2-isopropyl-2-phenyl-5-(phenethylamino)valeronitriles, being substituted on the phenethylamino moiety. The compounds of the present invention are structurally related to verapamil, which is known to be a mixed ion channel blocker for the calcium and potassium channels.

Immunosuppression is a technique necessary in the fields of organ and bone-marrow transplantation. Immunosuppression agents are used for prophylaxis and treatment for organ rejection in allogeneic transplants (transplants between individuals of the same species, but not genetically identical). Such therapy helps prolong survival of transplants of the kidney, liver, and heart, as well as to a lesser extent transplants of the pancreas, heart-lung transplants and bone-marrow transplants. Such immunosuppression is necessary in order to alter immune system reactions to the presence of the foreign organ or tissue, that will normally result in rejection and wholesale destruction by the hosts' immune system of the transplanted organ or tissue.

Cyclosporin A is a particularly effective immunosuppressor agent in the prophylaxis and treatment of organ rejection. Cyclosporin A is one of a family of compounds and is a hydrophobic cyclic undecapeptide produced by the fungus Tolypocladium inflatum gams. Other cyclosporins include cyclosporin B and cyclosporin G. Cyclosporin A suppresses humoral immunity as well as cell-mediated reactions. Cyclosporin A is indicated for organ rejection in kidney, liver, heart, pancreas, bone-marrow and heart-lung transplants. Cyclosporin A is also useful in the treatment of Crohn's disease, Graves ophthalmopathy, severe psoriasis, aplastic anemia, multiple-sclerosis, alopecia areata, and penphigus and penphigoid, dermatomyositis, polymyositis, Behcet's disease, uveitis, pulmonary sarcocidiosis, biliary cirrhosis, myasthenia gravis and atopic dermatitis.

Unfortunately, cyclosporin A can cause renal and hepatoxicity, causing significant liver or kidney damage for patients receiving organ transplants, including damage to the transplanted kidney itself if the procedure is a kidney transplant. Therefore, it is an object of the present invention to provide a method of administering class of compounds which, when administered to a mammal, will effect immunosuppression and yet show significantly lower toxicity than the administration of cyclosporin . It is a further object of the present invention to provide a class of compounds which, when administered to a mammal concurrently with a cyclosporin or other immunosuppressants, will act in a synergistic manner to lower the dosage of a cyclosporin or other immunosuppressant needed to achieve a given level of immunosuppression, thus decreasing the likelihood of causing toxicity caused by the cyclosporin or other immunosuppressant. Other immunosuppressants include levamisol, corticosteroids, non-steroidal anti-inflammatory drugs (NSAIDS), certain prostaglandins and the macrolide FK-506 (Fujisawa).

The present invention provides a novel method of administering new substituted 2-isopropyl-2-phenyl-5-(phenethylamino) valeronitriles, which show efficacy as immunosuppressing agents by virtue of their ability to inhibit interleukin- production, thereby inhibiting T-lymphoblasts and thus T-cell proliferation. Inhibition of T-cell proliferation will in turn block activation of cytotoxic-T lymphocytes, a key step in the normal mammalian immune response.

The present invention further provides that the compounds here have activity and utility as systemic antihypertensives, site-selective vasodilating activity useful in the treatment of renal ischemia and cerebral ischemia. The mechanism of action for the immunological activities of the compounds of the present invention is thought to be independent of the cardiovascular calcium channel blocking activity of the compounds.

The invention relates to the use of compounds of the general formula I:

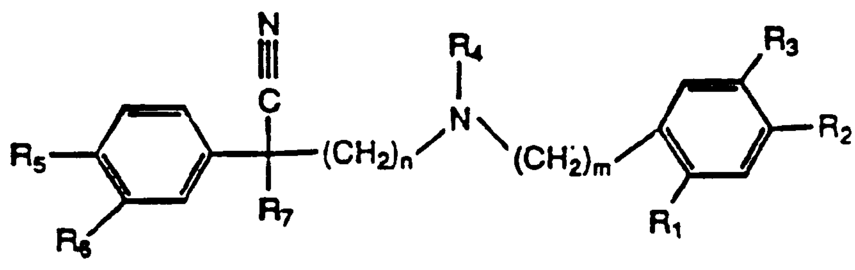

and the pharmaceutically acceptable salts thereof, wherein

$R_1$ is; hydroxy; substituted or unsubstituted alkyl, alkenyl or alkynyl of 1 to 10 carbon atoms, wherein said substituent is hydroxy or keto; alkylcarbonyl of 1 to 10 carbon atoms;
alkoxycarbonyl of 1 to 10 carbon atoms; or
alkylamido of 1 to 10 carbon atoms;
$R_2$, $R_3$, $R_5$ and $R_6$ are independently hydroxy; or
alkoxy of 1 to 10 carbon atoms; and
$R_4$ and $R_7$ are independently hydrogen, alkyl, alkenyl or alkynyl of 1 to 10 carbon atoms;
m is an integer of from 1 to 2, inclusive; and
n is an integer of from 1 to 5, inclusive for preparing a medicament for immunosuppression of a mammal. A more preferred embodiment of the present invention relates to administration of novel compounds of the general formula II:

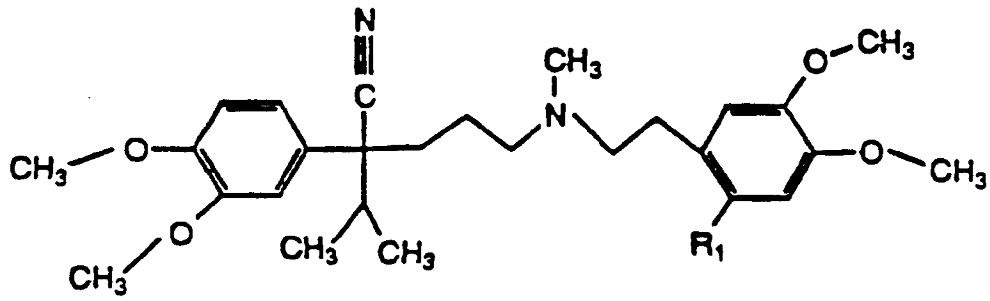

and the pharmaceutically acceptable salts thereof, wherein
$R_1$ is hydrogen;
hydroxy; substituted or unsubstituted alkyl, alkenyl, or alkynyl of 1 to 10 carbon atoms, wherein said substituent is keto or hydroxy; alkylcarbonyl of from 1 to 10 carbon atoms;
alkoxycarbonyl of 1 to 10 carbon atoms; or
alkylamido of 1 to 10 carbon atoms.

The compounds and pharmaceutical compositions thereof are useful in the immunosuppressing methods of the invention. The invention further provides for administration of dosage unit forms adapted for oral, topical and parenteral administration. Also provided for in this invention is the administration of the pharmaceutically acceptable salts of the compounds, for immunosuppression.

As used herein, the expressions "alkyl", alkenyl and "alkynyl" are defined to include straight or branched carbon-carbon linkages of 1 to 10 carbon atoms with 1 or more degrees of unsaturation.

The terms "alkylcarbonyl" and "alkoxycarbonyl" is defined to include alkyl, alkenyl or alkynyl moieties as defined above.

The term "alkylamido" is defined to include alkyl, alkenyl or alkynyl moieties as defined above.

The term "pharmaceutically acceptable salts" refers to non-toxic salts of the compounds of this invention which are generally prepared by reacting the free base with a suitable organic or inorganic acid or by reacting the acid with a suitable base. Representatives salts include the following salts:

| | |
|---|---|
| Acetate | Lactobionate |
| Benzenesulfonate | Laurate |
| Benzoate | Malate |
| Bicarbonate | Maleate |
| Bisulfate | Mandelate |
| Bitartrate | Mesylate |
| Borate | Methylbromide |
| Bromide | Methylnitrate |
| Calcium edetate | Methylsulfate |
| Camsylate | Mucate |
| Carbonate | Napsylate |
| Chloride | Nitrate |
| Clavulanate | Oleate |
| Citrate | Oxalate |
| Dihydrochloride | Pamoate (Embonate) |
| Edetate | Palmitate |
| Edisylate | Pantothenate |
| Estolate | Phosphate/diphosphate |
| Esylate | Polygalacturonate |
| Fumarate | Salicylate |
| Gluceptate | Stearate |
| Gluconate | Subacetate |
| Glutamate | Succinate |
| Glycollylarsanilate | Sulfate |
| Hexylresorcinate | Tannate |
| Hydrabamine | Tartrate |
| Hydrobromide | Teoclate |
| Hydrochloride | Tosylate |
| Hydroxynaphthoate | Triethiodide |
| Iodide | Valerate |
| Isethionate | |
| Lactate | |

As used herein, the term "immunosuppression" shall mean the artificial controlled diminution or prevention of the mammalian immune response.

The term "ischemia" shall mean deficiency of blood to a part of the body, due to constriction of one or more blood vessals.

The term "hypertension" shall mean persistently high arterial blood pressure.

The term "pharmacologically effective amount" shall mean that amount of the drug or pharmaceutical agent that will elicit the biological or medical response of a tissue, system or animal that is being sought by a researcher or clinician.

The term "immunosuppressive agent" shall include cyclosporins A, B and G, corticosteroids, levamisol, non-steroidal anti-inflammatory drugs and those prostaglandins exhibiting immunosuppressive activity.

Compounds used in practicing the immunosuppressant method of the invention can be prepared readily according to the following reaction schemes or modifications thereof using readily available starting materials, reagents and conventional synthesis procedures. In these reactions, it is also possible to make use of variants which are themselves known, but are not mentioned in greater detail.

EXAMPLE 1

Scheme I

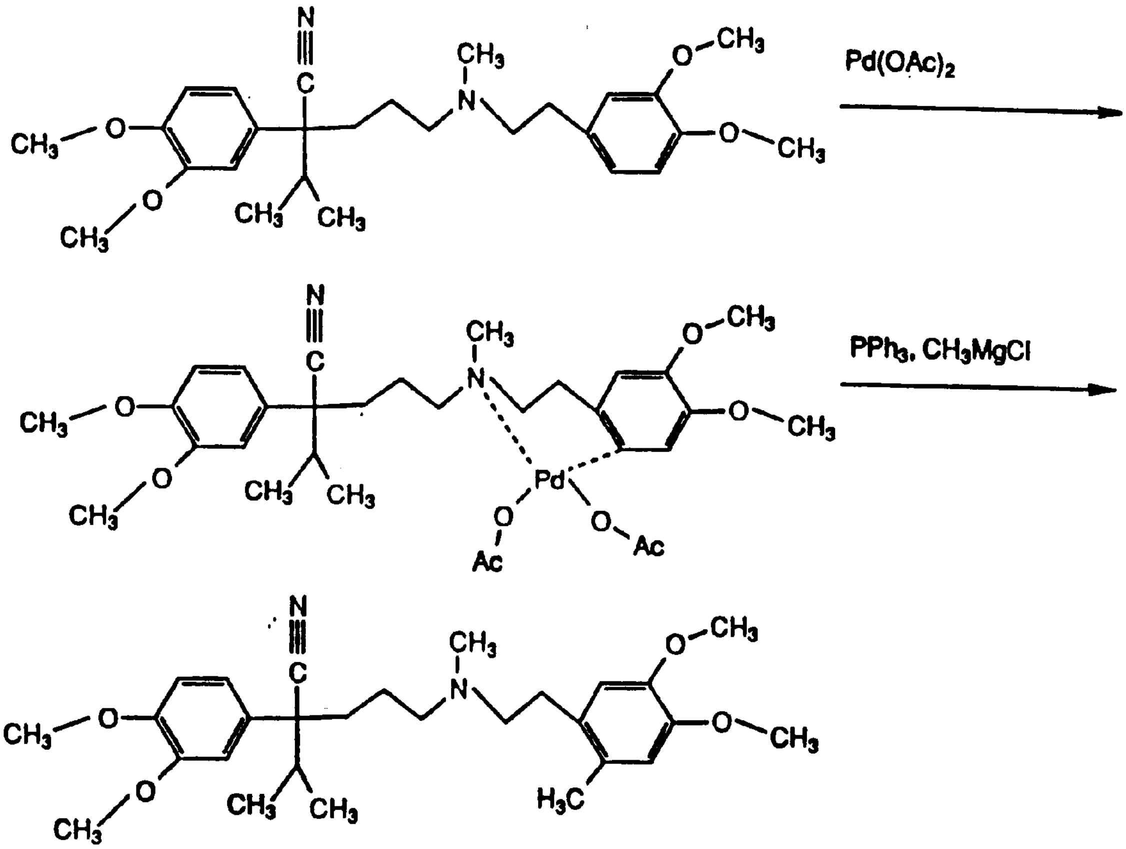

Scheme II

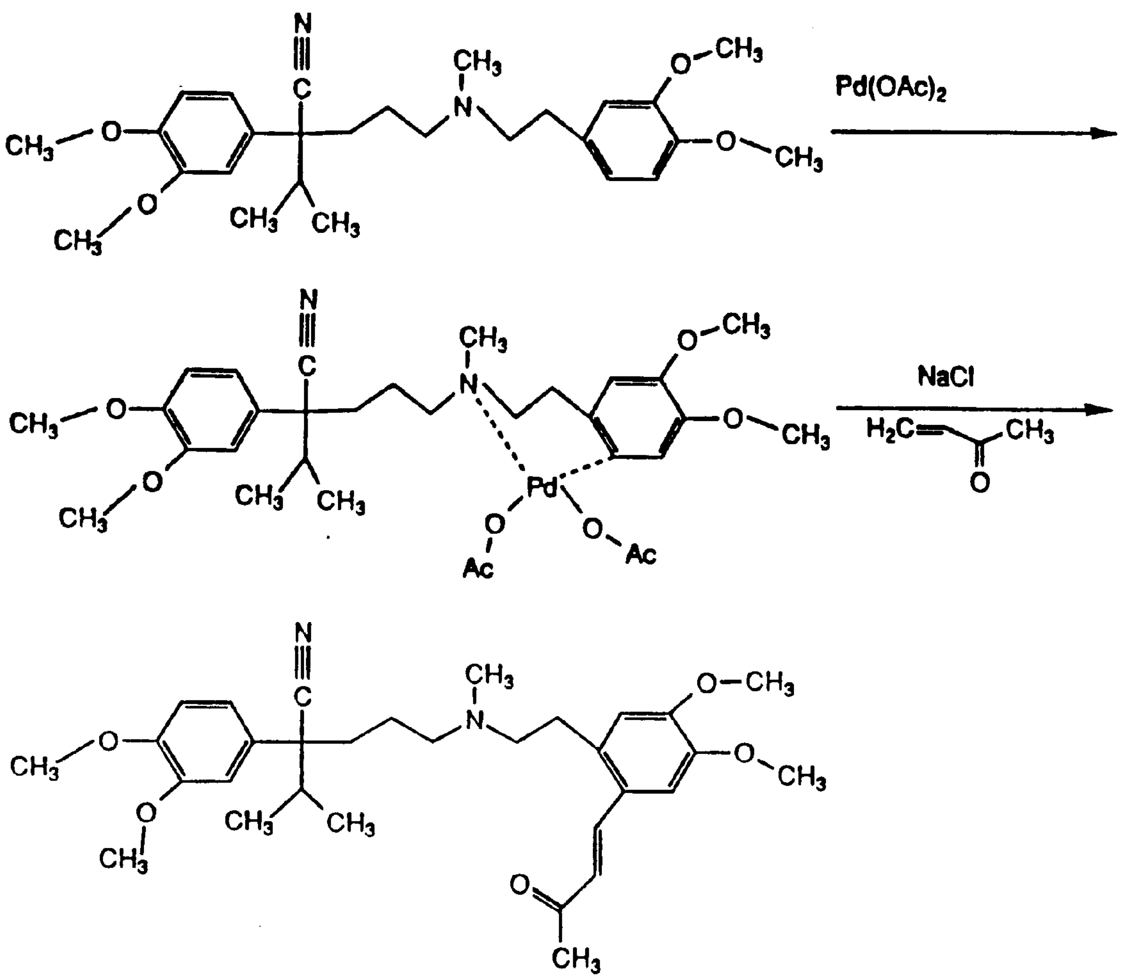

Scheme III

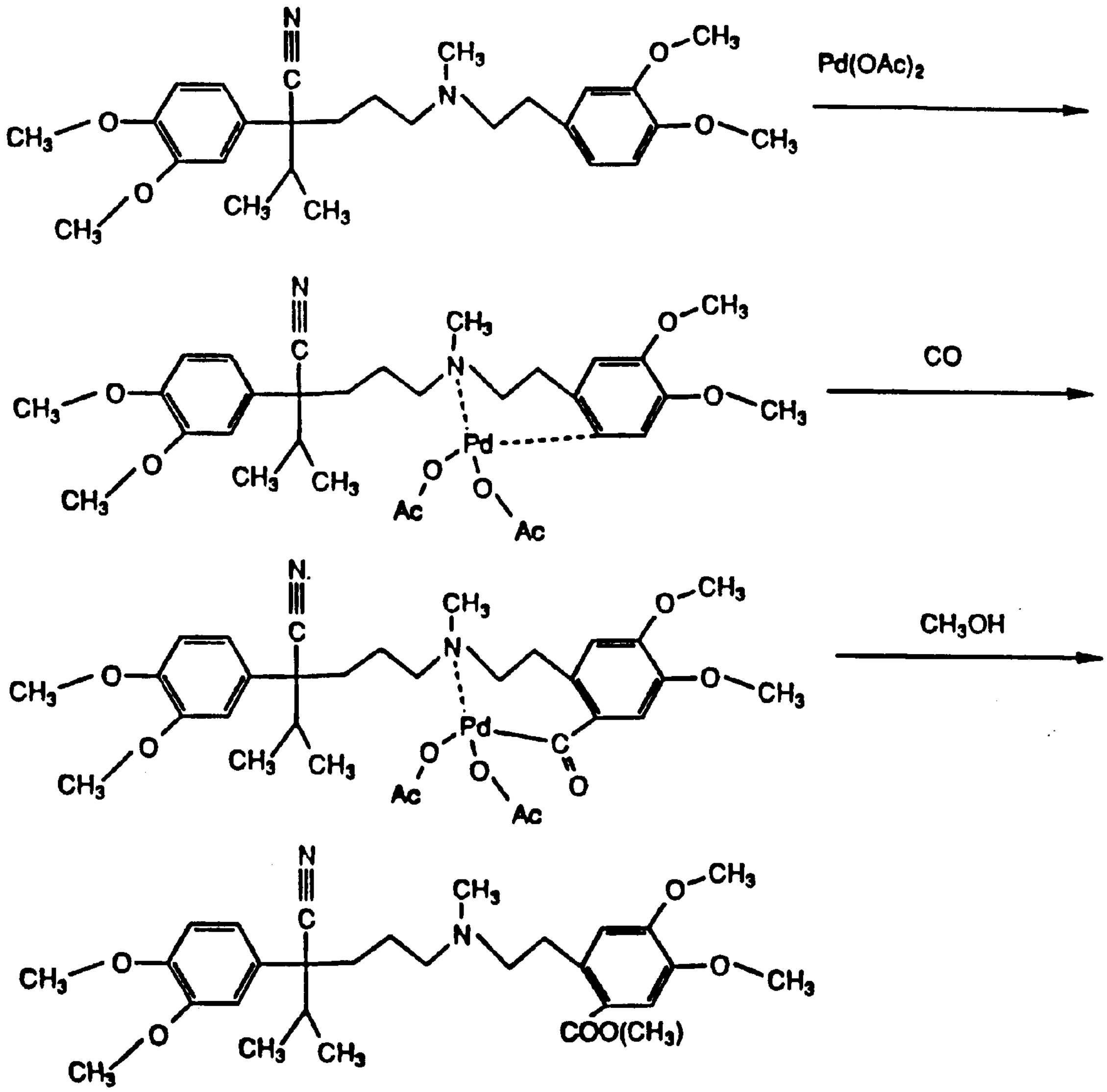

Scheme IV

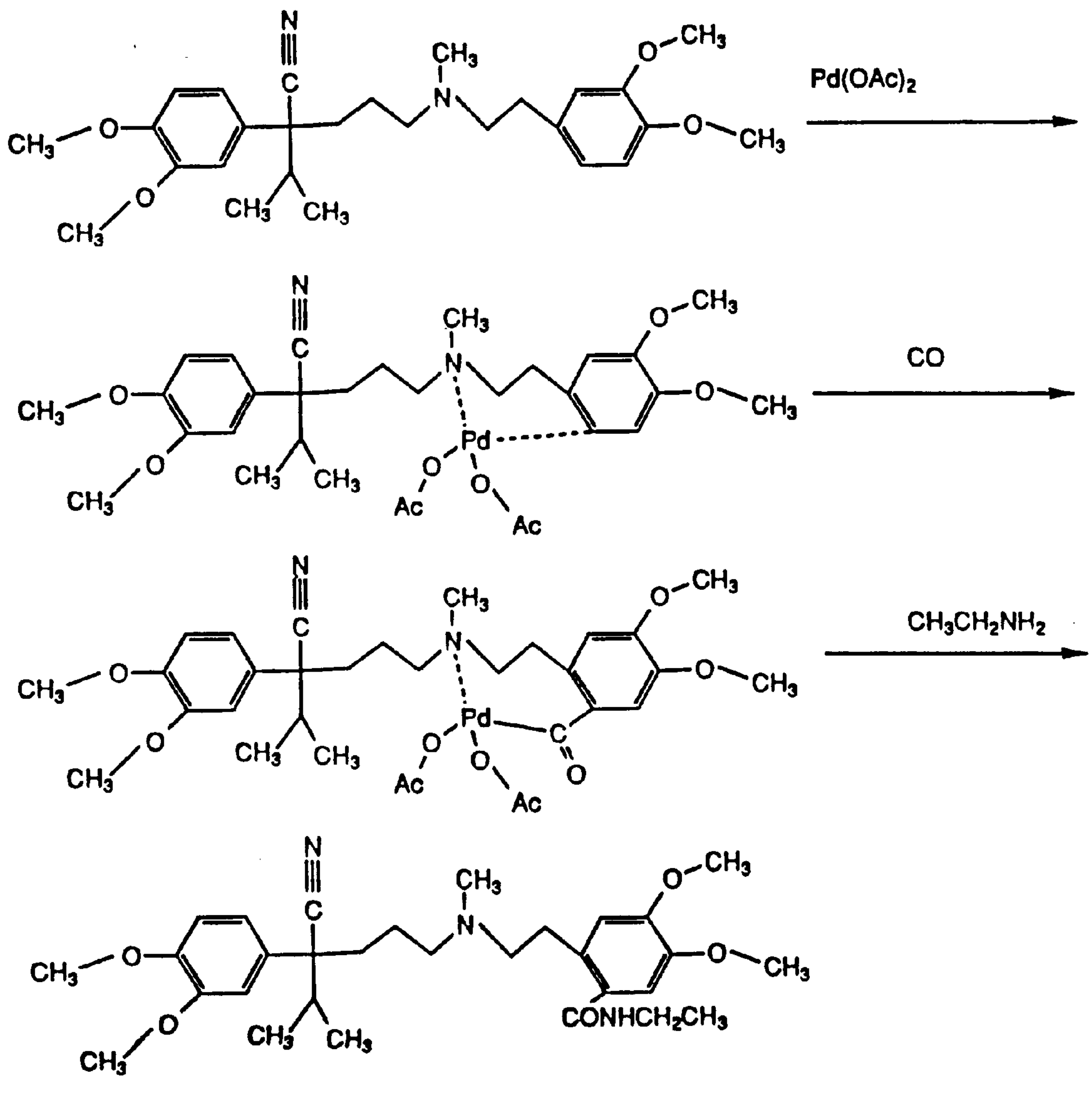


In the above schemes, Ac denotes a $COCH_3$ moiety: Ph denotes a phenyl moiety.

In achieving immunosuppression, the compounds of the present invention can be administered in such oral dosage forms as tablets, capsules, pills, powders, granules, elixers, tinctures, suspensions, syrups and emulsions. Likewise, they may also be administered in intravenous, intraperitoneal, subcutaneous or intramuscular form, all using forms known to those of ordinary skill in the pharmaceutical arts.

In general, the preferred form of administration is oral. An effective but non-toxic amount of one of the compounds claimed in the novel method can be employed as an immunosuppressor in the inhibition of the mammalian immune response or in treatment of hypertension, renal ischemia or cerebral ischemia. An alternative embodiment of the present invention is a novel method of administration of an effective non-toxic

amount of one or more of said compounds concurrently with effective but less than previously prescribed amounts of a cyclosporin in a regimen of immunosuppression therapy. The dosage regimen of the present invention is selected in accordance with a variety of factors including the type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to achieve immunosuppression.

Dosages of the compounds utilized in the methods of the present invention, when used for the indicated effects, will range between about 0.1 milligram per kilogram of body weight per day (mg/kg/day) to about 1,000 mg/kg/day and preferably 1.0-100 mg/kg/day. Advantageously, compounds utilized in the method of the present invention may be administered in a daily single dose or the total daily dosage may be administered in divided doses of 2, 3 or 4 times daily.

Furthermore, preferred compounds utilized in the present invention can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous throughout the dosage regimen.

In the methods of the present invention, the compounds described herein in detail can form the active ingredient, and are typically administered in admixture with suitable pharmaceutical diluents, excipients or carriers (collectively referred to herein as "carrier" materials) suitably selected with respect to the intended form of administration, that is, oral tablets, capsules, elixirs, tinctures, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methylcellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugar such as glucose or beta-lactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The compounds utilized in the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine or phosphatidylcholine.

The compounds utilized in the present invention may also be delivered by the use of monoclonal antibodies as individual carriers to which the compound molecules are coupled. The compounds utilized in the present invention may also be coupled with soluble polymers as targetable drug carriers. Such polymers can include polyvinylpyrrolidone, pyran copolymer, polyhydroxypropylmethacrylamide-phenol, polyhydroxyethylaspartimide-phenol, or polyethyleneoxide-polylysine substituted with palmitoyl residues. Furthermore, the compounds may be coupled to a class of biodegradable polmers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross-linked or amphipathic block copolymers of hydrogels.

The compounds utilized in the method of this invention exhibit immunosuppression activity useful in the treatment of rejection of transplanted organs. The test procedures employed to measure this activity of the compounds are described below.

EXAMPLE 2

NMR spectra were obtained on either a Varian XL-200 or a GTE-QE 300 spectrometer in solvent as indicated with $(CH_3)_4Si$ as the internal standard. Infrared spectra were recorded on a Perkin-Elmer 685 spectrometer in $CHCl_3$.

General procedure for preparation of 2-(3,4-dimethoxyphenyl)-2-isopropyl-5-[3,4-dimethoxy-(6-substituted phenethyl)methylamino]valeronitrile:

To a stirred solution of 2.5 mmole verapamil in 250 ml benzene was added palladium (II) acetate 30 mmole and was stirred for four days. It was then transferred to a gasometric apparatus (R. F. Heck, J.A.C.S.,

83, 1097 (1961), flushed with carbon monoxide (CO) and at room temperature with the addition of nucleophiles (alcohol, amine, Grignard reagents). After four hours, the reaction mixture was filtered and the solvent removed under reduced pressure. The residue was chromatographed on silica gel.

EXAMPLE 3

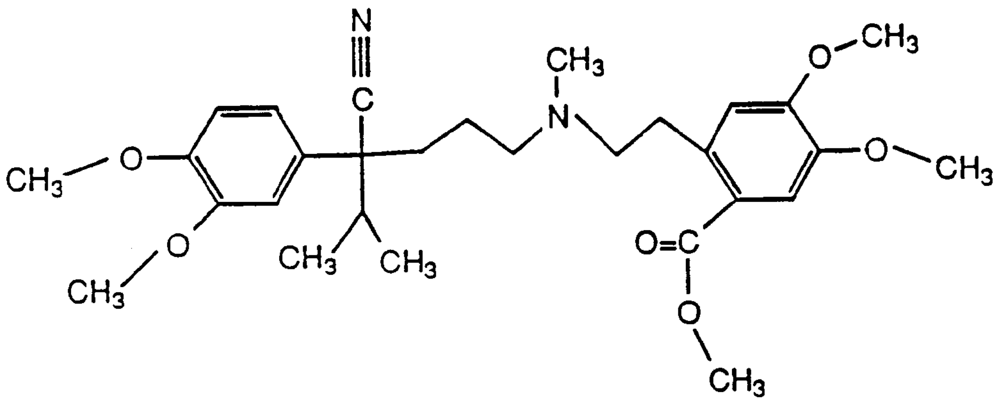

The addition of $CH_3OH$ to the carbonylated palladium complex obtained the title compound.

| | Cald. | Fd. | |
|---|---|---|---|
| C | 67.95 | 67.01 | |
| H | 7.86 | 8.12 | $C_{29}H_{40}N_2O_6$ |
| N | 5.46 | 5.24 | |

NMR δ 0.79(3H, d, J=6.6H)1.18(3H, d, J=6.6H)1.19(1H, m) 1.55-2.05(m, 3H), 2.23(3H, S), 2.25-2.75(7H, m)6.5-6.8 (m, 4H)7.35(S, 1H), 3.80, (S, 3H)3.78(S, 3H), 3.88 (S, 3H)3.89(S.3H)

IR 1718cm$^{-1}$ 41%

EXAMPLE 4

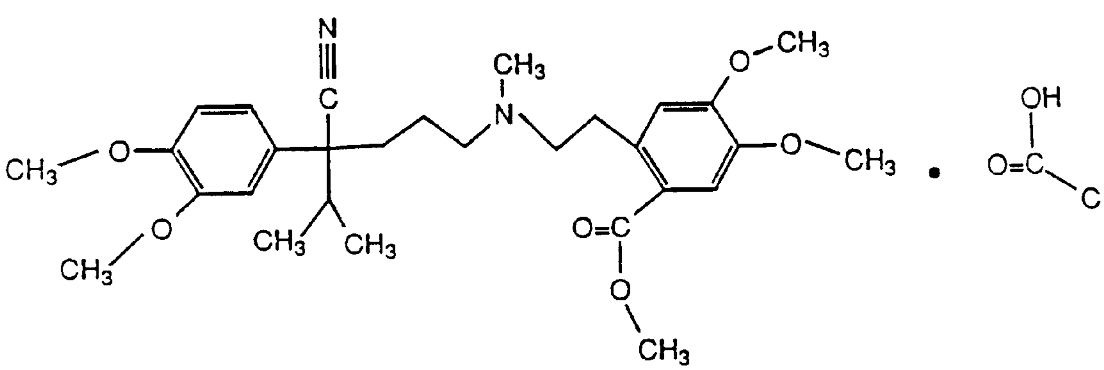

The title compound was prepared from the addition of citric acid in ether to the etheral solution of the product of Example 3 in ether. The precipitate was filtrated and dried.

| | Cald. | Fd. | |
|---|---|---|---|
| C | 59.65 | 60.11 | |
| H | 6.86 | 7.10 | $C_{35}H_{48}N_2O_{13}$ |
| N | 3.97 | 3.99 | |

By way of the above described method, one of ordinary skill in the art can obtain the 1, 2,3-propanetricarboxylic acid salt or the 2,3 dihydroxybutanedioic acid salt.

EXAMPLE 5

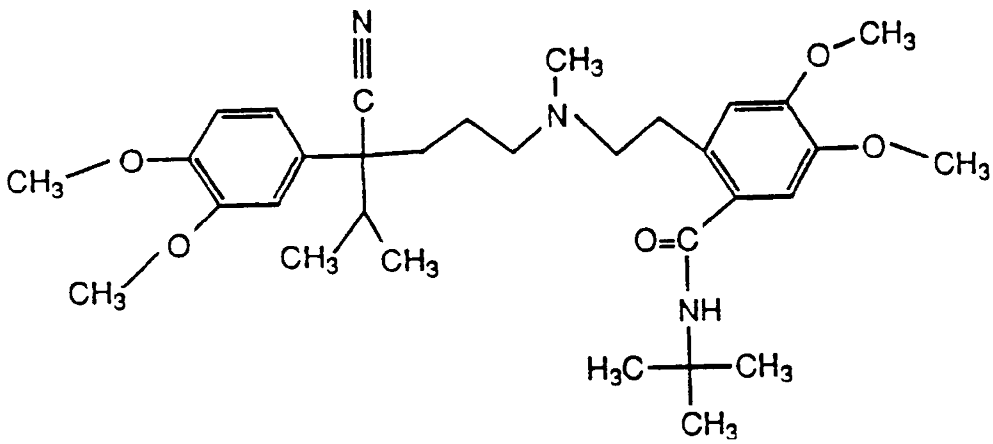

The addition of t-butylamine to the carbonylated palladium complex obtained the title compound. The citric acid salt of the title compound was obtained from the addition of saturated citric acid to the etheral solution of the free base. The precipitate collected was dried. Obtained: 32%.

Alternatively, addition of methyl amine instead of t-butylamine would yield the corresponding methylamide.

| | Cald. | Fd. | |
|---|---|---|---|
| C | 62.03 | 62.72 | |
| H | 6.16 | 7.46 | $C_{38}H_{55}N_3O_{12}$ |
| N | 5.71 | 6.08 | |

NMR 0.75ppm, (d, 3H, J=6.6H), 1.16(d, 3H, J=6.6H)
1.46ppm(S, 9H), 2.8(S, 3H, N-me)3.81(S, 3H)
3.83(S, 9H), 6.97-7.03(m, 4H)7.36(S, 1H)

IR 1715cm$^{-1}$(citrate), 1640cm$^{-1}$, 2210cm$^{-1}$

By the method of example 4, the citric acid salt, the 1,2,3-tricarboxylic acid salt or the 2,3-dihydroxybutanedioic acid salt can be obtained.

EXAMPLE 6

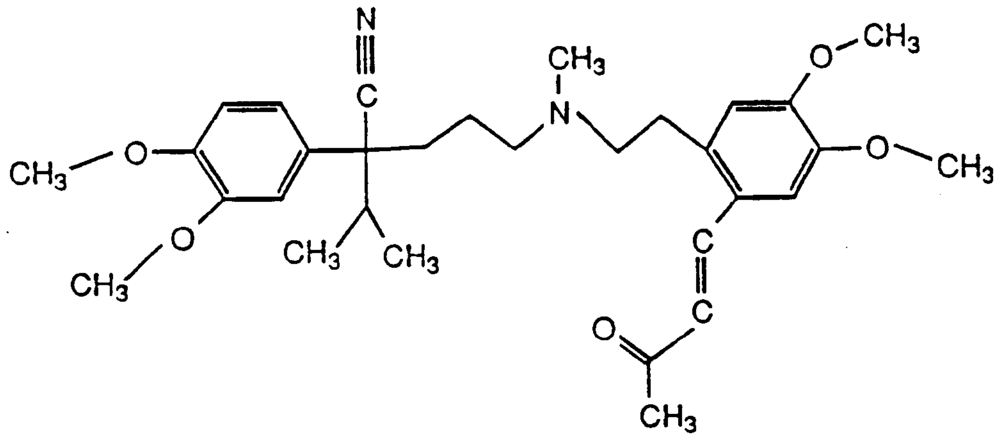

4 g Verapamil (8.8 mmole) in 100 ml of benzene was added to 2.2 g of $Pd(OAc)_2$, and stirred for four days at room temperature. A saturated solution of NaCl in 100 ml of $H_2O$ and 96 ml of acetone was added to the resulting solution, which was stirred at room temperature for 15 minutes. Solvent was then removed under reduced pressure.

Then was added 100 ml of toluene, 25 ml of triethylamine, and the mixture was heated at 120° for 2 hours, and then allowed to cool to room temperature. The organic phase was filtrated, washed with water, dried, filtrated again and solvent removed.

After chromatograph, the title compound was obtained (3.2g, 80%) m.p. 119-121 (from ether).

| | Cald. | Fd. |
|---|---|---|
| C | 71.24 | 71.57 |
| H | 8.10 | 8.30 |
| N | 5.36 | 5.25 |

NMR (ppm), 0.79(d, 3N, J=6.6H), 1.18(d, 3H, J=6.6H)
2.22(S, 3H), 2.36(S, 3H), 6.56(d, 1H, J=15H)
6.68(S, 1H), 6.80-6.92(m, 2H), 6.86(S, 1H)
7.08(S, 1H), 7.76(d, 1H, J=15H)
3.87(S, 3H), 3.88(S, 3H), 4.00(S, 3H), 4.01(S, 3H)

IR 3410cm$^{-1}$, 2150cm$^{-1}$ 1659cm$^{-1}$, 1639cm$^{-1}$,
1593cm$^{-1}$, 1509cm$^{-1}$

EXAMPLE 7

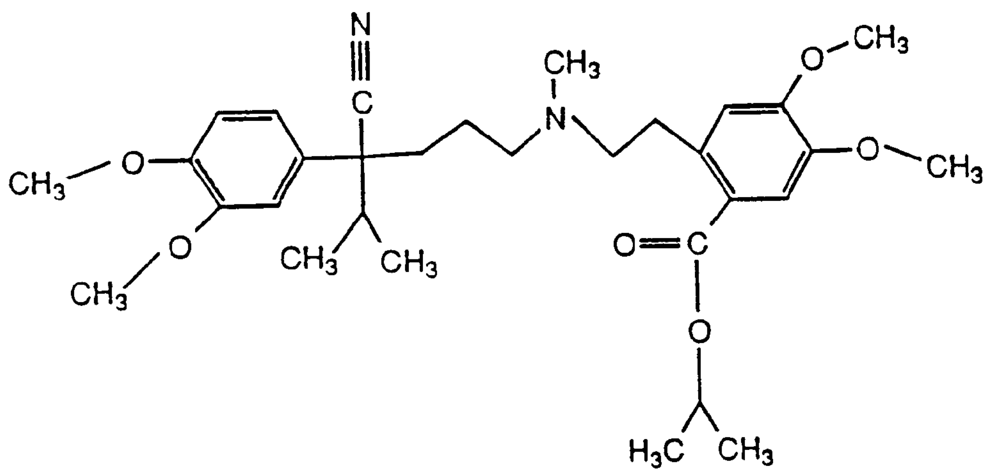

Isopropanol was added to the carbonylated palladium complex. Obt: 40%

| | Cald. | Fd. |
|---|---|---|
| C | 68.86 | 68.34 |
| H | 8.20 | 8.32 |
| N | 5.18 | 4.92 |

NMR, δ 0.79(3H, d, J=6.6H), 1.18(34, d, J=6.6H)
1.38(6H, d, J=6.6H), 2.24(3H, S, 3H)
3.80, (S, 3H)3.82(S, 3H)3.85(S,3H)3.90(S, 3H)
5.24(Septet J=6.6H, 1H), 6.73(5, 1H)
6.90(m, 3H) 7.05(S, 1H)
IR, $(CuCl_3)$ $1714cm^{-1}$

EXAMPLE 8

A. Effects on mitogen induced T-cell proliferation.

Using methods well known to those of ordinary skill in the art, the compounds utilized in the present invention were found to inhibit phytohemagglutinin (PHA) induced T-cell proliferation in a dose-related fashion, over a range of from 10 to 100 uM. Relative order of potency tested was the compound of Example 4 (the 1,2,3-propanetricarboxylic acid salt) > the compound of Example 5 (the 1,2,3-propane tricarboxylic acid salt) > the compound of Example 5 (the 2,3-dihydroxybutanedioic acid salt).

A similar dose-dependant relationship and order of relative potency in inhibiting T-cell proliferation could be observed using interleukin-2 dependant PHA-induced T-lymphoblast cells as targets of the various compounds. The addition of exogenous human recombinant IL-2 to either PHA-induced blast cells or to fresh cultures of PHA and peripheral blood mononuclear leukocytes (PBMC) did not overcome the anti-proliferative effects of the compounds.

B. Synergism of the compounds utilized in the present invention with cyclosporin A (CsA) in inhibiting mitogen induced T-cell proliferation.

The compounds utilized in the present invention synergize with CsA in blocking T-cell proliferation in vitro. Using methods well known to those of ordinary skill in the art, PHA stimulated PBMC were cultured in a constant dose of each compound tested and with decreasing doses of CsA, starting with the minimum dose of

CsA that totally blocks proliferation (125 ng/ml). CsA doses as low as 6 ng/ml were utilized. The concentration of the compound of Example 4 (the 1, 2, 3-propane tricarboxylic acid salt) was kept constant at 5 uM, whereas the concentrations of the compound of Example 5 (the 1, 2, 3-propanetricarboxylic acid salt) and of the compound of Example 5 (the 2, 3-dihydroxybutanedioic acid salt) were kept at 25 uM. Synergism was evidenced by a super-additive inhibition of the mitogen-induced T-cell proliferation, by the antiproliferative effects mediated by CsA and the tested compounds. Addition of the compounds utilized in the present invention shifted the $IC_{50}$ of CsA by 3 to 4-fold.

C. Effects of the compounds utilized in the present invention on T-cell activation gene expression.

CsA has several characteristic effects upon expression of various de novo transcriptional events in PHA stimulated cultures. Transcription of interleukin-2 (IL-2) messenger RNA (mRNA), but not IL-2 receptor mRNA is blocked in mitogen stimulated human PBMCs. Total RNA was extracted from human PBMCs from unstimulated, mitogen stimulated, and mitogen stimulated drug treated (100 uM) cultures. Transcription was analyzed by Northern blot techniques; RNA was hybridized to radiolabeled cDNA probes for IL-2 and p55 IL-2-receptor mRNA (TAC, CD25). The compound of Example 4 (the 1,2,3-propantricarboxylic acid salt) and the compound of Example 5 (the 1,2,3-propanetricarboxylic acid salt) at 100 uM abolished accumulation of IL-2 mRNA, while IL-2 receptor mRNA was, at most, slightly diminished. Translation and surface expression of the p55 IL-2-receptor protein (CD25) was only slightly inhibited by either compound. Although CD25 expression was slightly reduced by high concentrations of the two compounds, surface expression of the TAC (CD25) protein was clearly detectable by FACS analysis using anti CD25 monoclonal antibodies. In addition, activation induced surface expression of HLA-DR antigens was not inhibited by these agents. In contrast, in keeping with the abscence of IL-2 mRNA, IL-2 protein could not be detected in the culture supernatants by use of an ELISA system.

The compounds utilized in the method of the present invention, have unexpected, surprising and unobvious activity as immunosuppressants. Likewise, their synergistic effect with CsA was unexpected and unobvious. Their dose-lowering synergism with CsA means that in therapeutic immunosuppression they can be expected to avoid side effects of hypotension, bradycardia and nephrotoxicity.

EXAMPLE 9

The compound which is the product of Example 1, scheme 1, was found to be site-selective for brain and kidney tissue in causing decreases in vascular resistance through calcium channel blockade activity.

Under ether anesthesia, the jugular vein, femoral artery and left ventricle of adult male SHR were cannulated. After a 3-5 hour recovery period, animals were treated with either test compound or placebo. Ten minutes was allowed for steady state conditions to be reached. Arterial pressure was then monitored via the femoral cannula. Immediately after this measurement, the reference blood sample taken and radioactively-labled microspheres were injected into the left ventricle. Rats were then sacrificed; organs were removed, weighed and counted. Cardiac output was computed. Percent cardiac output distribution per gram tissue, flow per gram tissue, and resistance times gram tissue were calculated for the following organs: brain, heart, kidneys, splanchnic organs (liver plus pancreas, spleen and G.I. tract), skeletal muscle and skin. The tested compound was administered as an I.V. bolus of 1.0 mg/kg. Differences in mean values between treatment and control groups were analyzed by a one-way one variable analysis and least significant difference test. The subject compound caused a decrease of vascular resistance to the brain:

| | |
|---|---|
| Example 1, Scheme 1 | 149mm Hg min. g/ml |
| Control, DMSO | 240mm Hg min. g/ml |

$p < 0.05$

and to the kidneys:

| | |
|---|---|
| Example 1, Scheme 1 | 22.4mm Hg min. g/ml |
| Control, DMSO | 37.6mm Hg min. g/ml |

$p < 0.05$

The therapeutic usefulness of such site-selectivity is in treatment of cerebral or renal ischemia.

EXAMPLE 10

Calcium antagonist activity is measured in vitro by measuring the $pA_2$ (the negative logarithm of the apparent dissociation constant of the drug molecule for its receptor) in isolated aortic smooth muscle of the spontaneously hypertensive rat. The greater the $pA_2$, the greater the calcium antagonism. A compound is considered active as a vascular calcium antagonist if the $pA_2$ is 6.0 or greater.

The $Ca^{++}$ ion plays an essential role in induction and maintenance of vascular smooth muscle contractility. In $K^+$-depolarized vascular smooth muscle, calcium antagonists block the entry of $Ca^{++}$ into the cell, thereby inhibiting the contractions induced by $Ca^{++}$. The inhibition of $Ca^{++}$-induced contraction of vascular smooth muscle is used as a model to test compounds for vascular calcium antagonism.

Cardiovascular diseases such as arrhythmias, angina-pectoris, hypertension, peripheral vascular disease may be causally related to abnormalities in cellular handling of $Ca^{++}$ ions.

The excised aortic segment is mounted in a tissue bath containing modified Krebs solution. After depolarization of the tissue with $K^+$ (100 mM), $Ca^{++}$, in cumulative concentrations of $10^{-3}$M, $3.2\times10^{-3}$M and $10^{-2}$M, is injected into the bath to produce vascular smooth muscle contraction. The developed tension (g) is measured and control dose-response values are obtained. After 1 hr incubation with a test compound at $10^{-6}$M concentration, the same doses of $Ca^{++}$ are repeated. The log dose-response curves of control and after treatment are analyzed by linear regression.

| Example | $PA_2$ |
|---|---|
| 3 | 6.81 |
| 5 | 6.56 |
| 5 (1,2,3-propane-tri-carboxylic acid salt) | 6.57 |

EXAMPLE 11

Systemic blood pressure effects are assessed in vivo in an anesthetized spontaneously hypertensive rat. Test compound is administered intragastrically at $10^{-3}$M.

Initial mean arterial blood pressure (MAP) is measured directly via a previously implanted arterial catheter immediately before administration of the compound. Blood pressure readings are normally made 1, 2, 3, and 4 hours after administration of the test compound. This schedule of readings can be varied by request. A dose of test compound is rated active if the mean post-treatment blood pressure of treated rats is significantly different ($P \leqq 0.05$) than that of the concurrent placebo control group. Statistical comparisons are made using the non-paired Student's t test relative to the vehicle (placebo) treated control.

| Example | Decrease in MAP (mm Hg) |
|---|---|
| 5 | 18 |
| 5 (1,2,3-propane-tri-carboxylic acid salt) | 22 |

A most preferred group of compounds utilized in the novel methods of the invention consists of any or all of the following:

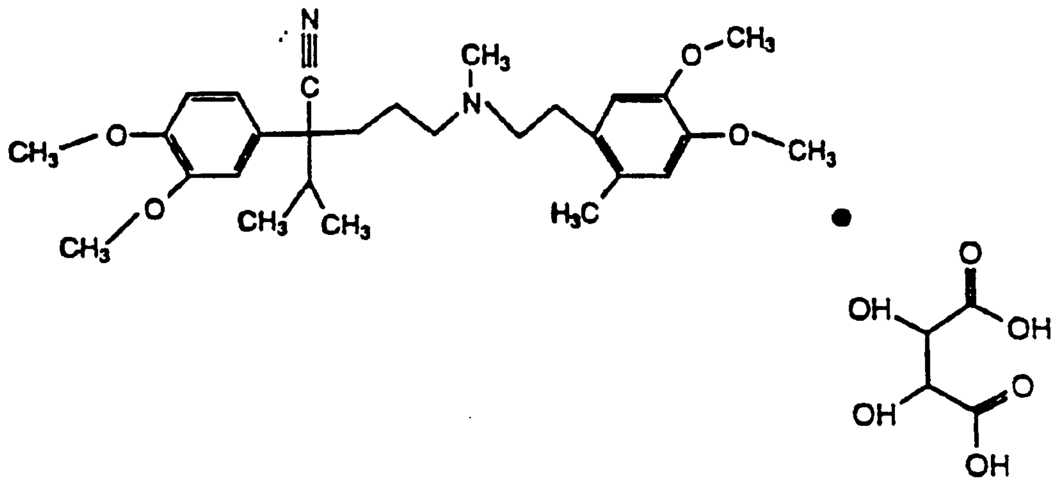

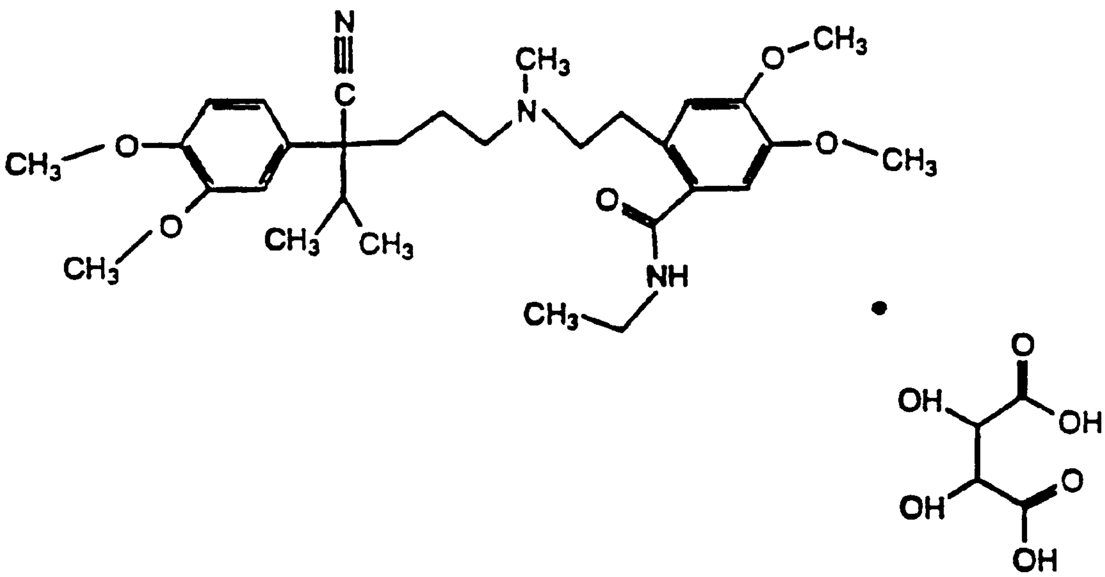

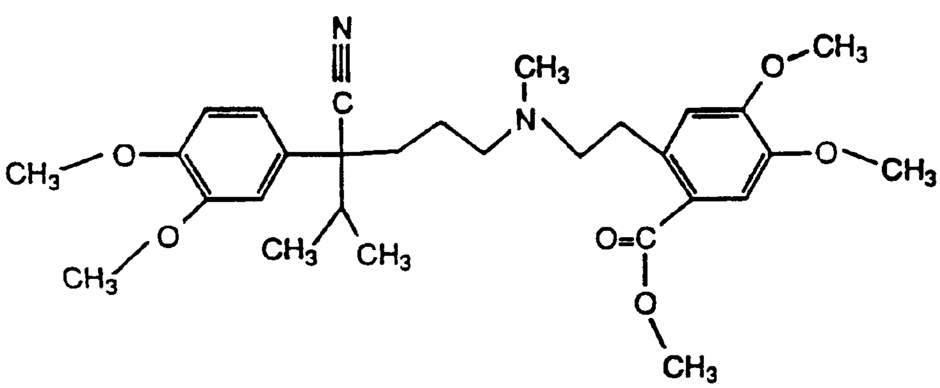

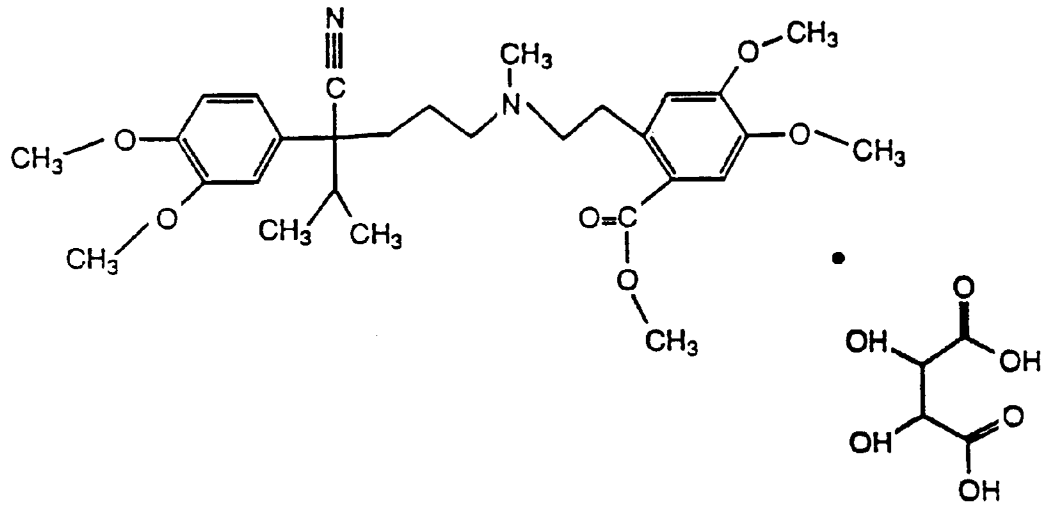

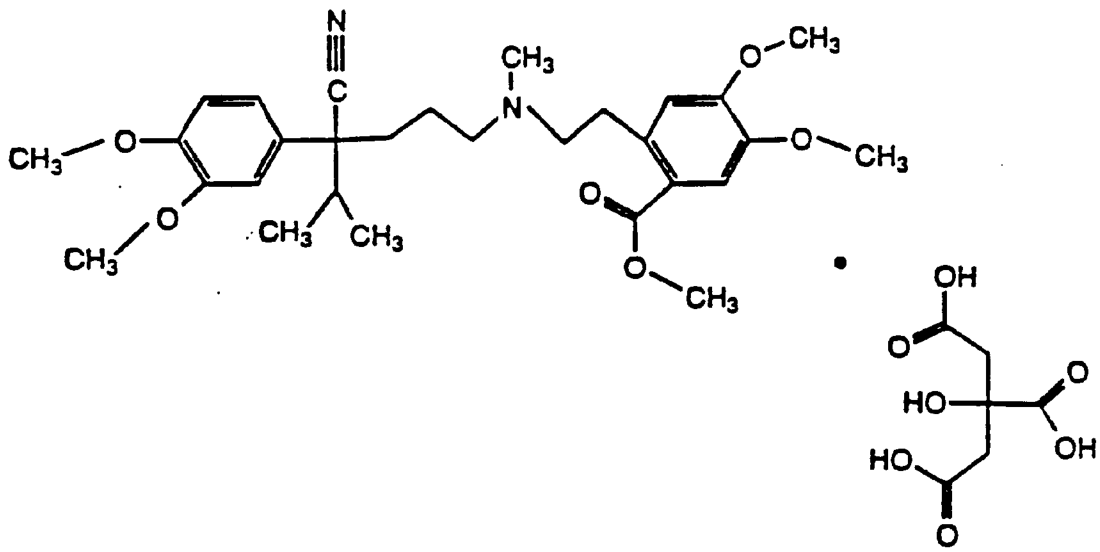

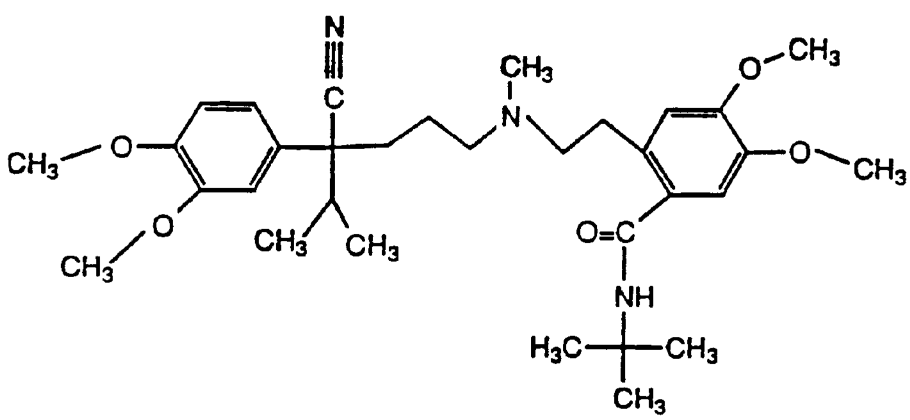

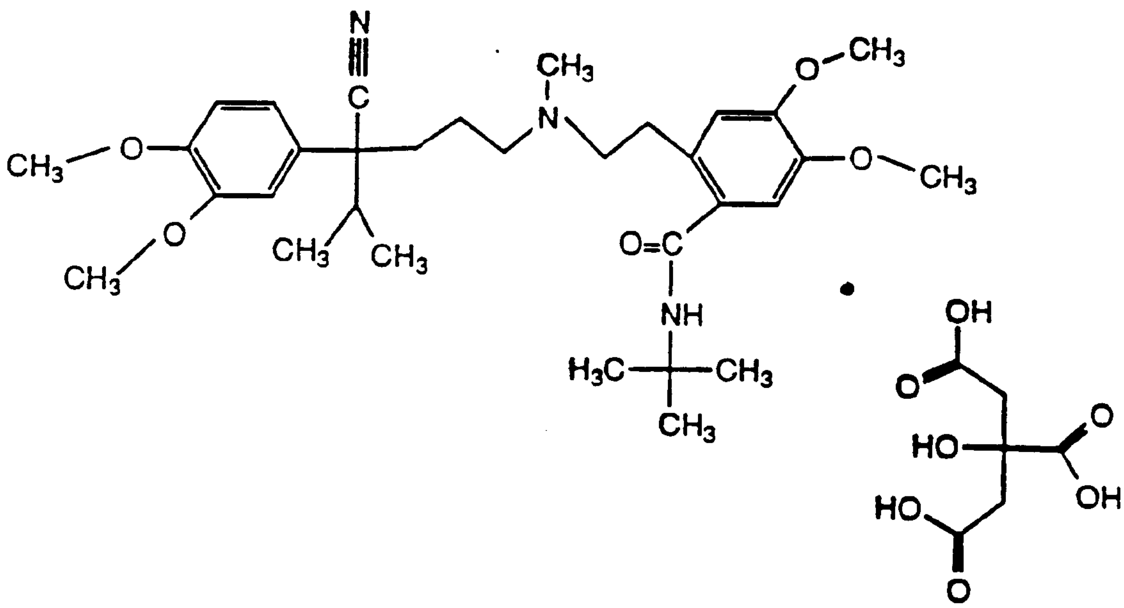

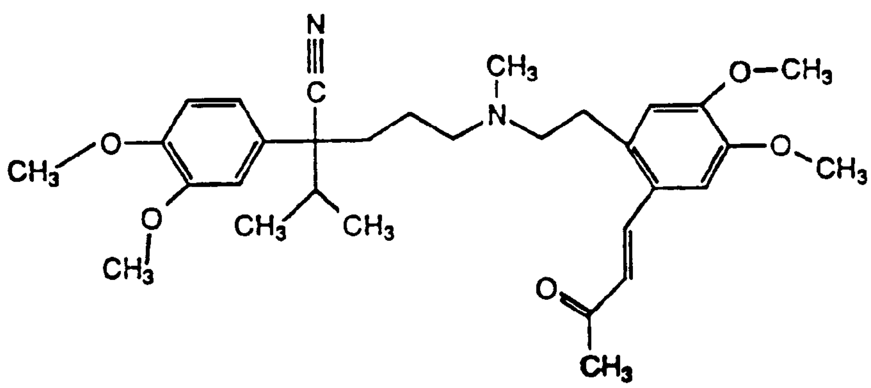

These compounds are not, however, to be construed as forming the only genus that is considered useful in the invention, and any combination of such compounds may itself form a genus.

Effective dosages other than the preferred ranges as set forth herein above may be applicable as a consequence of variations in the responsiveness of the mammal being treated for severity of organ transplant rejection, dosage-related adverse effects, if any, and analogous considerations. Likewise, the specific pharmacological responses observed may vary according to and depending upon the particular active compounds selected or whether there are present certain pharmaceutical carriers, as well as the type of formulation and mode of administration employed, and such expected variations or differences in the results are contemplated in accordance with the objects and practices of the present invention.

## Claims

1. Use of a compound of the structural formula:

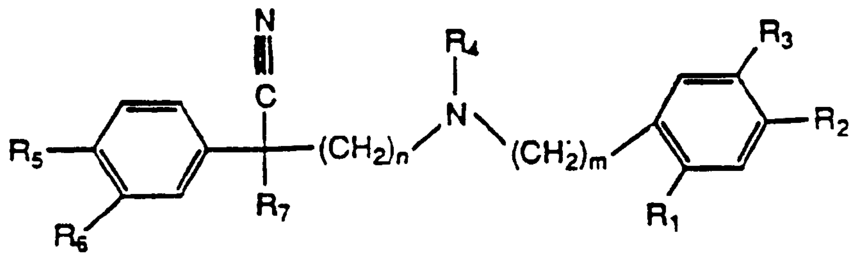

and the pharmaceutically acceptable salts thereof, wherein
$R_1$ is hydroxy;
substituted or unsubstituted alkyl, alkenyl, or alkynyl of 1 to 10 carbon atoms, wherein said substituent is hydroxy or keto; alkylcarbonyl of 1 to 10 carbon atoms;
alkoxycarbonyl of 1 to 10 carbon atoms; or
alkylamido of 1 to 10 carbon atoms;
$R_2$, $R_3$, $R_5$ and $R_5$ are independently hydroxy or
alkoxy of 1 to 10 carbon atoms; and
$R_4$ and $R_7$ are independently hydrogen;
alkyl, alkenyl or alkynyl of 1 to 10 carbon atoms;
m is an integer of from 1 to 2, inclusive; and
n is an integer of from 1 to 5, inclusive,
for preparing a medicament for immunosuppression of a mammal.

**2.** Use of a compound of the general formula:

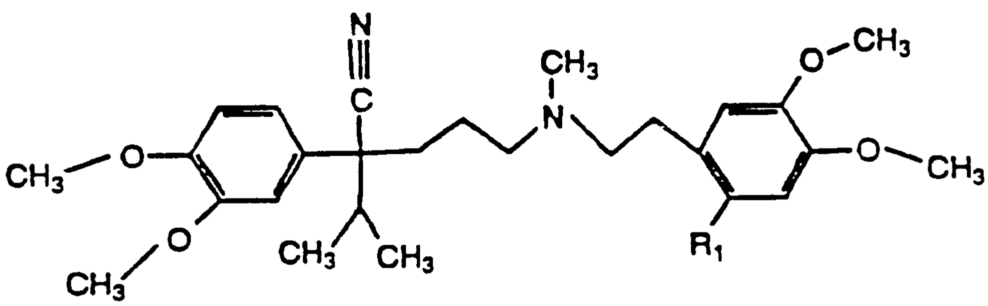

and the pharmaceutically acceptable salts thereof,
wherein, $R_1$ is hydroxy;
substituted or unsubstituted alkyl, alkenyl, or alkynyl of 1 to 10 carbon atoms wherein said substituent is keto or hydroxy;
alkylcarbonyl of from 1 to 10 carbon atoms;
alkoxycarbonyl of 1 to 10 carbon atoms; or
alkylamido of 1 to 10 carbon atoms,
for preparing a medicament for immunosuppression of a mammal.

**3.** Use according to Claim 1, wherein said compound having the structural formula:

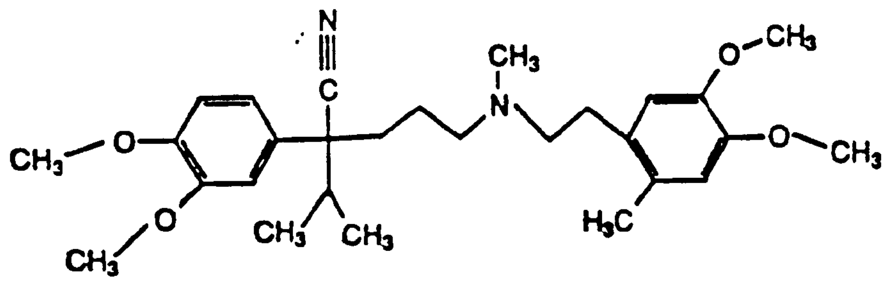

4. Use according to claim 1, wherein said compound having the structural formula:

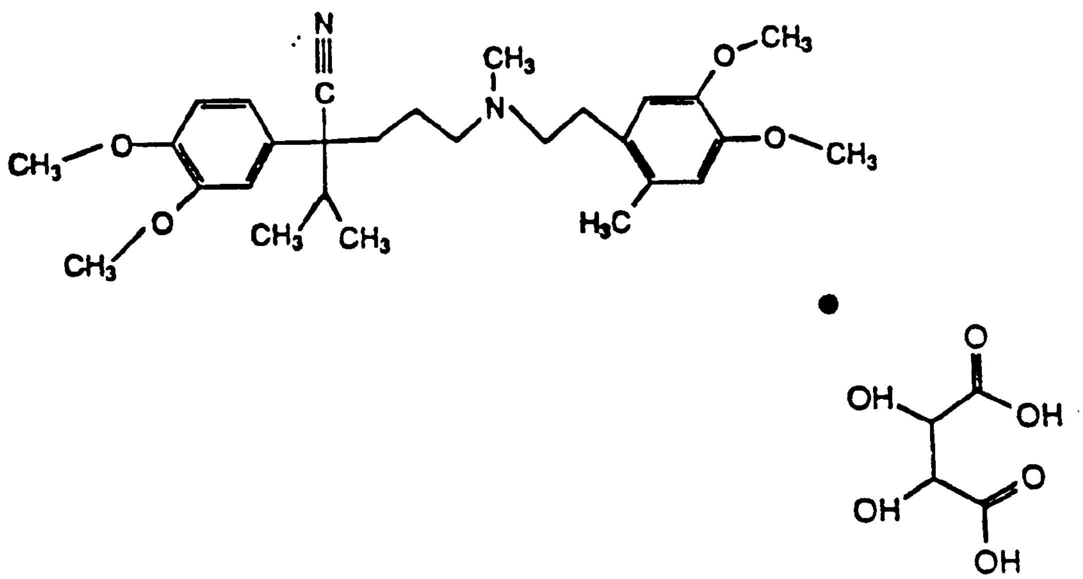

5. Use according to Claim 1, wherein the compound having the structural formula:

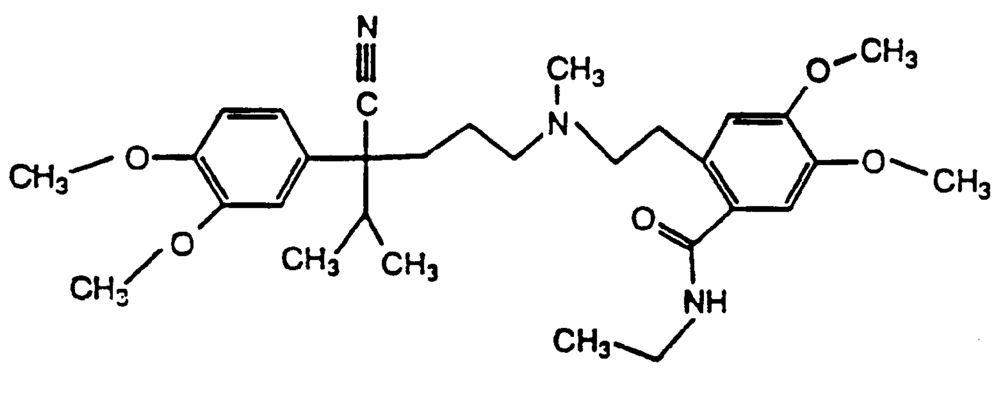

6. Use according to claim 1, wherein said compound having the structural formula:

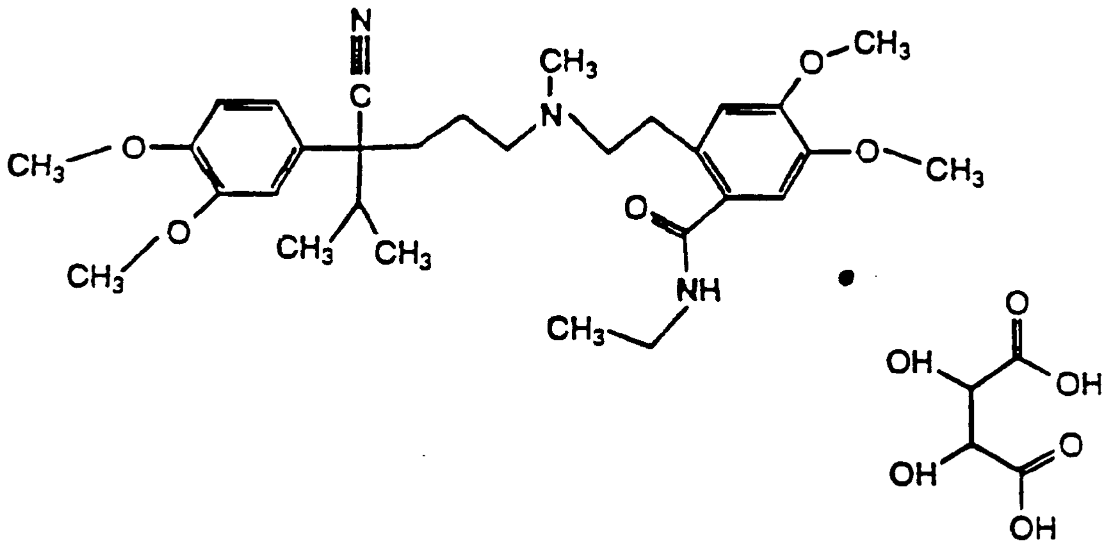

7. Use according to claim 1, wherein said compound having the structural formula:

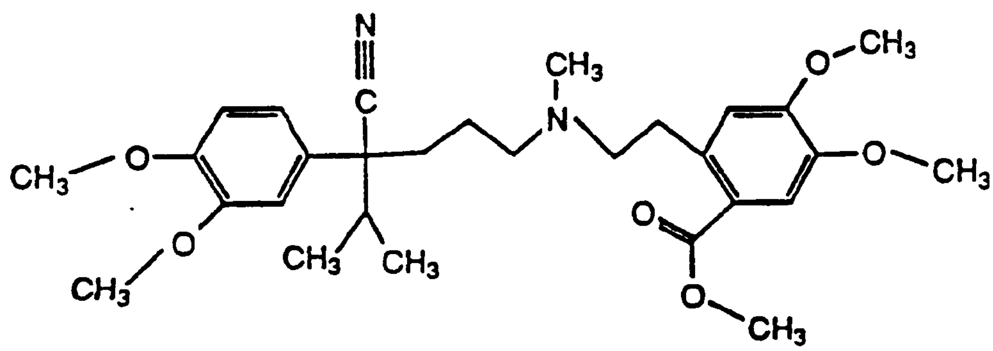

8. Use according to claim 1, wherein said compound having the structural formula:

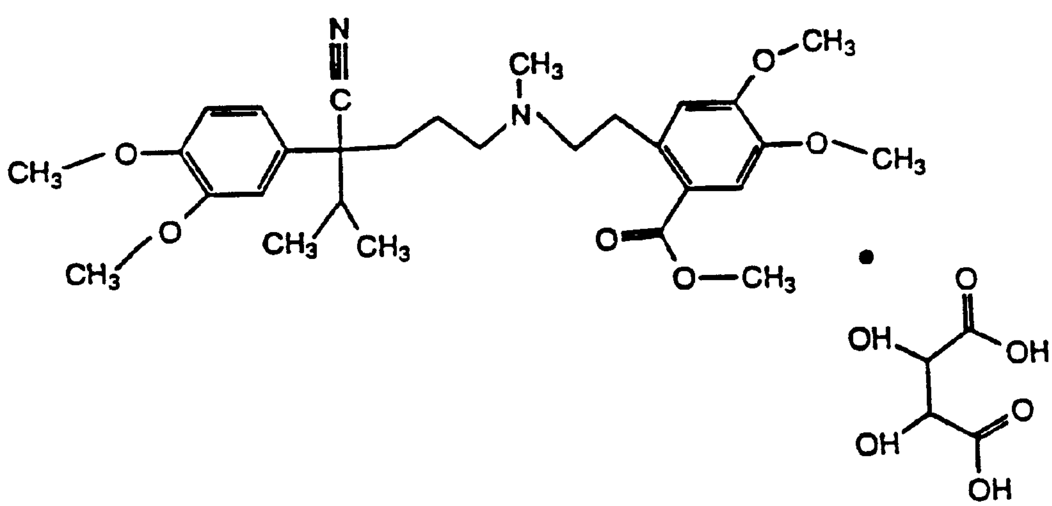

9. Use according to claim 1, wherein said compound having the structural formula:

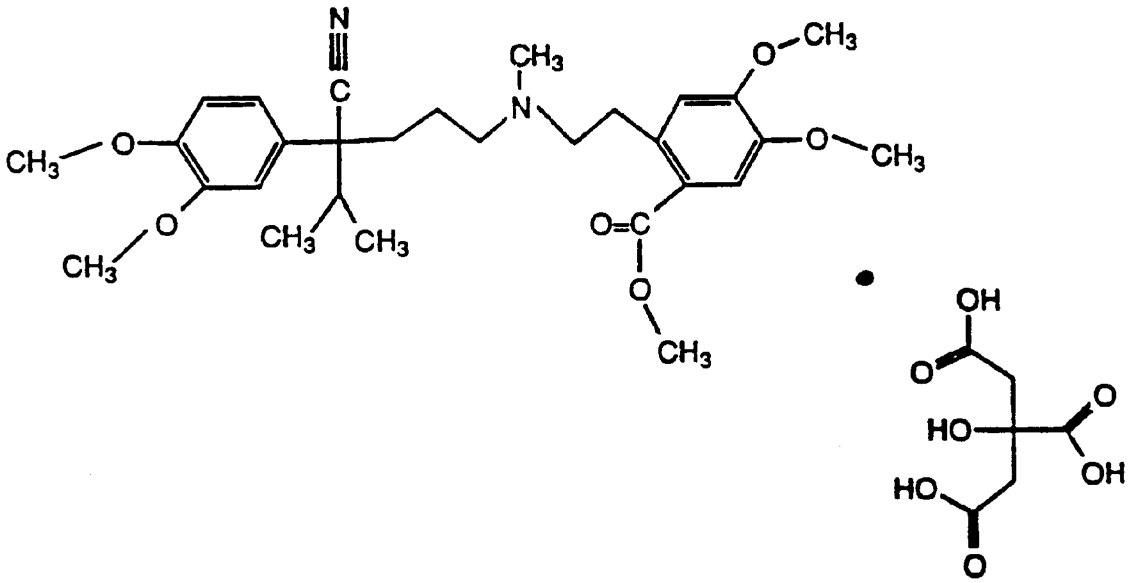

**10.** Use according to claim 1, wherein said compound having the structural formula:

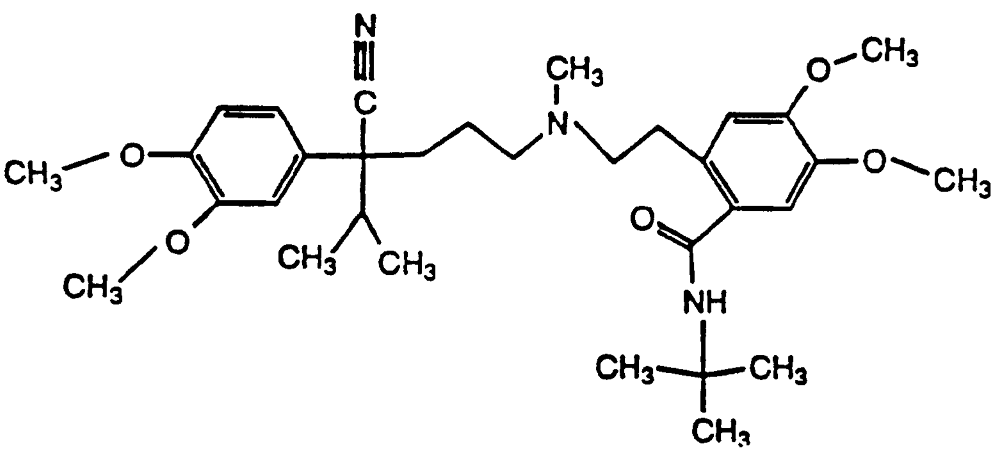

**11.** Use according to claim 1, wherein said compound having the structural formula:

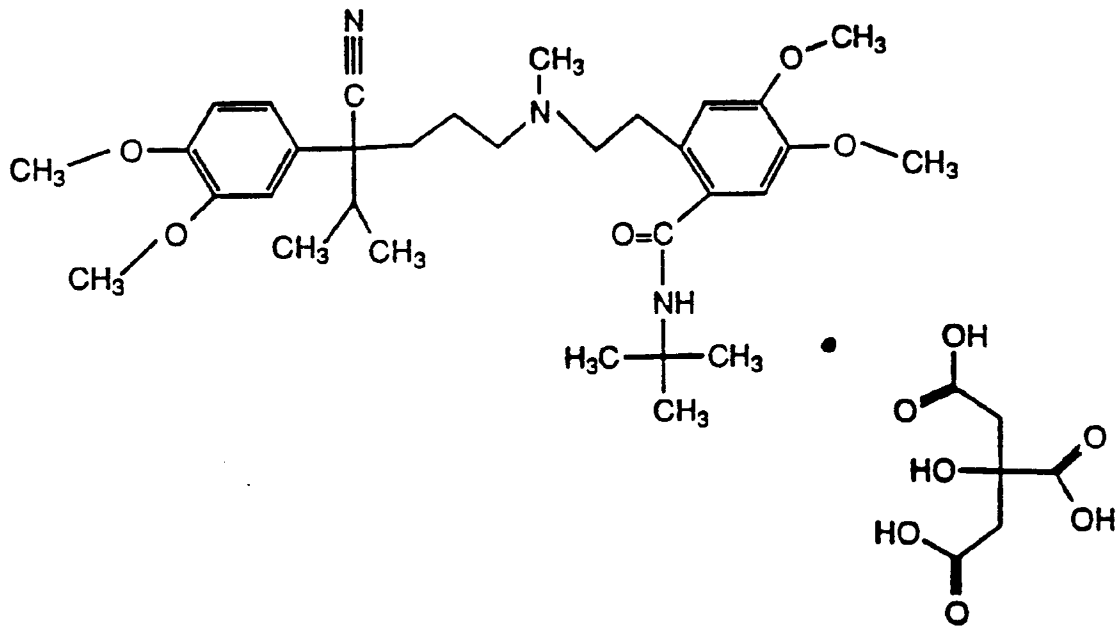

12. Use according to claim 1, wherein said compound having the structural formula:

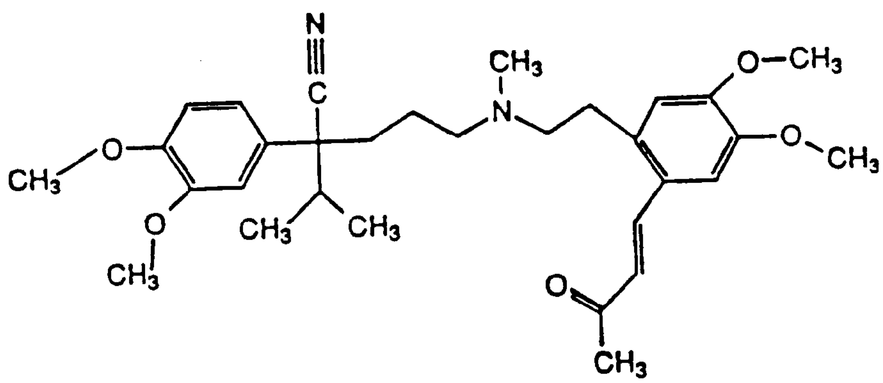

13. Use of a compound as defined in claim 1 for preparing a medicament for treating hypertension in a mammal.

14. Use of a compound as defined in claim 1 for preparing a medicament for treating renal ischemia in a mammal.

15. Use of a compound as defined in claim 1 for preparing a medicament for treating cerebral ischemia in a mammal.

**Patentansprüche**

1. Verwendung einer Verbindung der Formel:

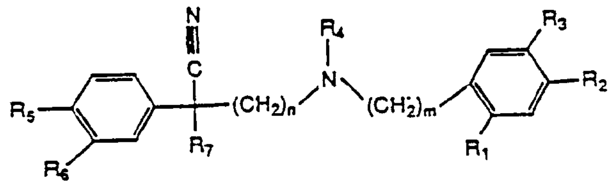

und deren pharmazeutisch verträglichen Salzen, worin
$R_1$ Hydroxy, einen substituierten oder unsubstituierten Alkyl-, Alkenyl- oder Alkinylrest mit 1 bis 10 Kohlenstoffatomen, worin der Substituent hierin Hydroxy oder Keto ist, oder Alkylcarbonyl mit 1 bis 10 Kohlenstoffatomen bedeutet,
Alkoxycarbonyl mit 1 bis 10 Kohlenstoffatomen oder Alkylamido mit 1 bis 10 Kohlenstoffatomen bedeutet;
$R_2$, $R_3$, $R_5$ und $R_6$ unabhängig voneinander Hydroxy oder Alkoxy mit 1 bis 10 Kohlenstoffatomen darstellen; und
$R_4$ und $R_7$ unabhängig voneinander Wasserstoff, Alkyl, Alkenyl oder Alkinyl mit 1 bis 10 Kohlenstoffatomen bedeuten;
m eine Zahl von 1 bis einschließlich 2 bedeutet und
n eine Zahl von 1 bis einschließlich 5 ist,
zur Herstellung eines Arzneimittels zur Immunsuppression in einem Säuger.

2. Verwendung einer Verbindung der allgemeinen Formel:

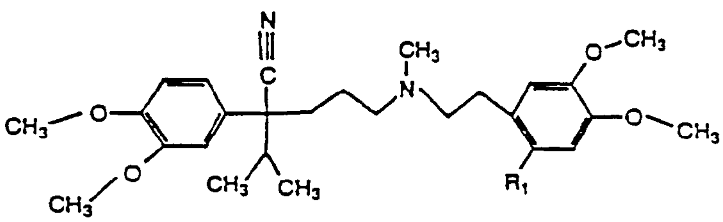

und deren pharmazeutisch verträglichen Salzen, worin
$R_1$ Hydroxy, eine substituierte oder unsubstituierte
Alkyl-, Alkenyl- oder Alkinylgruppe mit 1 bis 10 Kohlenstoffatomen, worin der Substituent Keto oder Hydroxy ist, bedeutet oder
Alkylcarbonyl mit 1 bis 10 Kohlenstoffatomen,
Alkoxycarbonyl mit 1 bis 10 Kohlenstoffatomen oder
Alkylamido mit 1 bis 10 Kohlenstoffatomen bedeutet,
zur Herstellung eines Arzneimittels zur Immunsuppression in einem Säuger.

3. Verwendung gemäß Anspruch 1, worin die Verbindung die Formel aufweist:

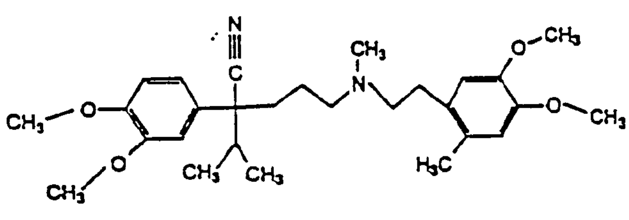

4. Verwendung gemäß Anspruch 1, worin die Verbindung die Formel aufweist:

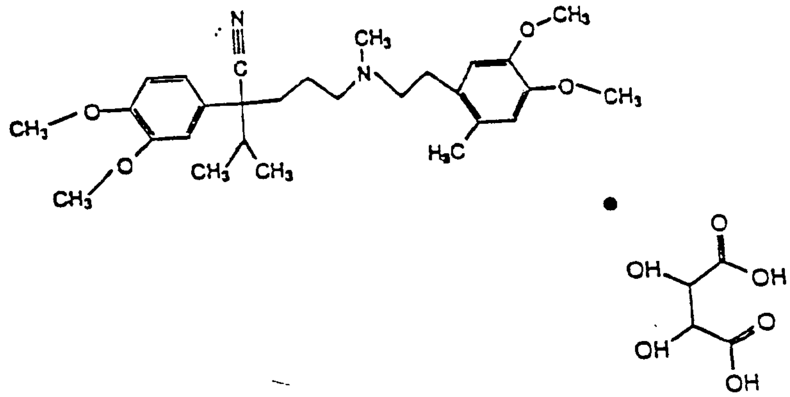

5. Verwendung gemäß Anspruch 1, worin die Verbindung die Formel aufweist:

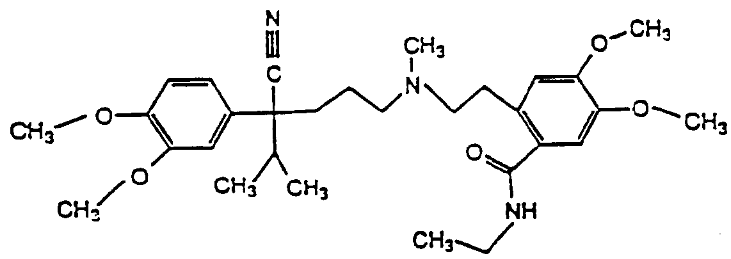

6. Verwendung gemäß Anspruch 1, worin die Verbindung die Formel aufweist:

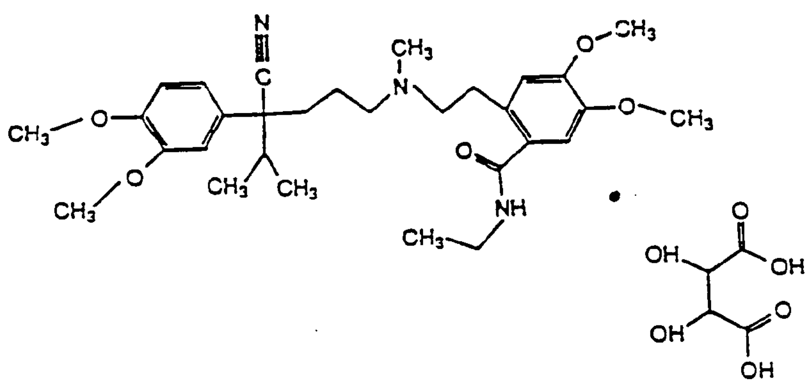

7. Verwendung gemäß Anspruch 1, worin die Verbindung die Formel aufweist:

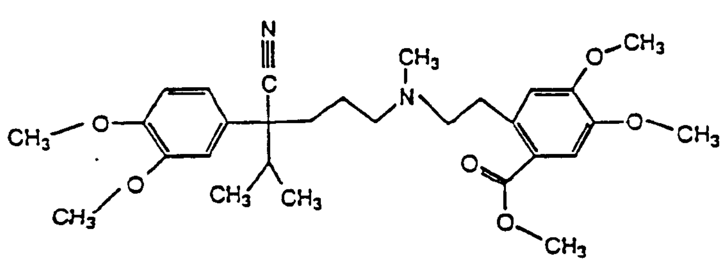

8. Verwendung gemäß Anspruch 1, worin die Verbindung die Formel aufweist:

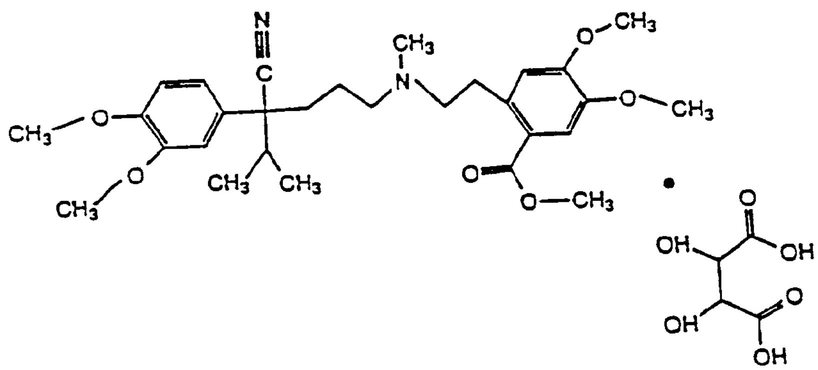

9. Verwendung gemäß Anspruch 1, worin die Verbindung die Formel aufweist:

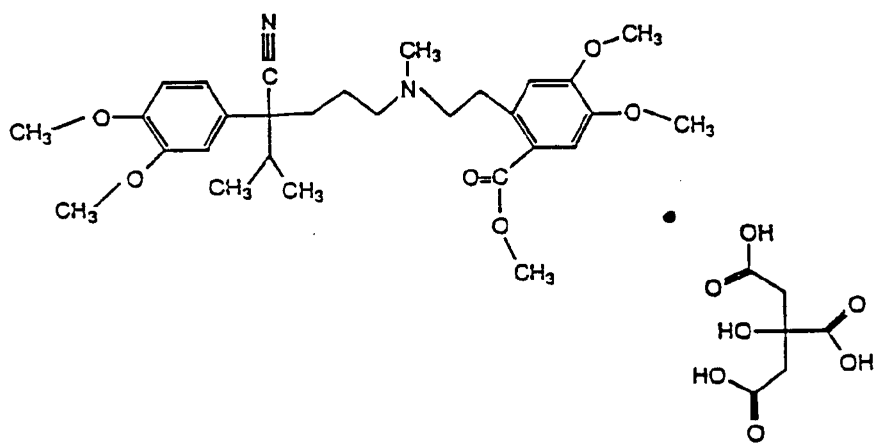

10. Verwendung gemäß Anspruch 1, worin die Verbindung die Formel aufweist:

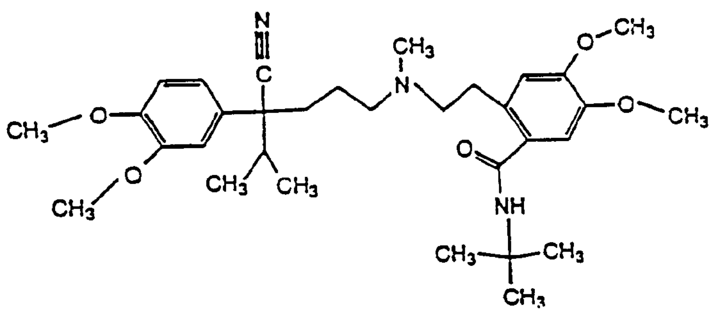

11. Verwendung gemäß Anspruch 1, worin die Verbindung die Formel aufweist:

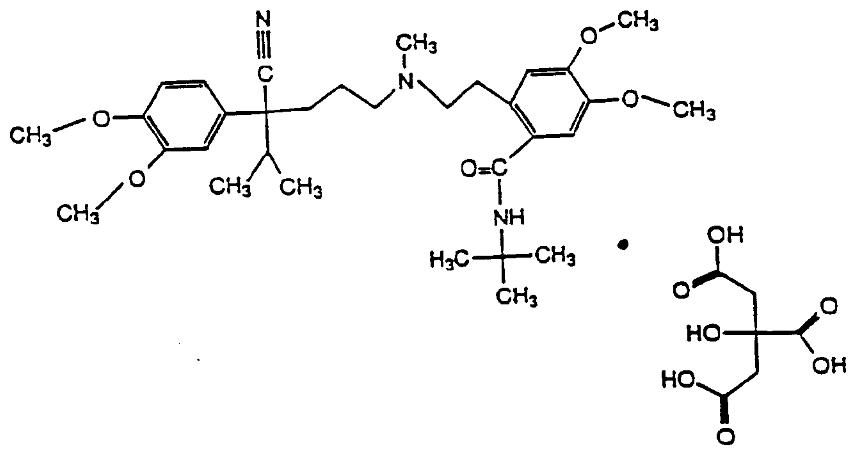

12. Verwendung gemäß Anspruch 1, worin die Verbindung die Formel aufweist:

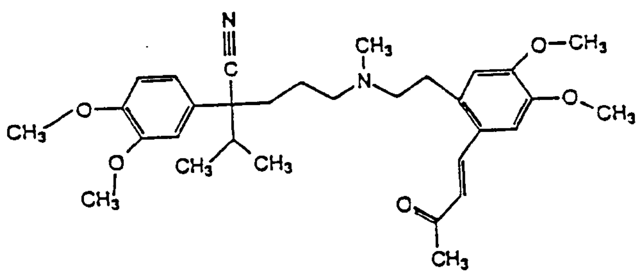

13. Verwendung einer Verbindung gemäß Anspruch 1 zur Herstellung eines Arzneimittels zur Behandlung von Bluthochdruck in einem Säuger.

14. Verwendung einer Verbindung gemäß Anspruch 1 zur Herstellung eines Arzneimittels zur Behandlung der renalen Ischämie in einem Säuger.

15. Verwendung einer Verbindung gemäß Anspruch 1 zur Herstellung eines Arzneimittels zur Behandlung der cerebralen Ischämie in einem Säuger.

## Revendications

1. Utilisation d'un composé de formule structurale :

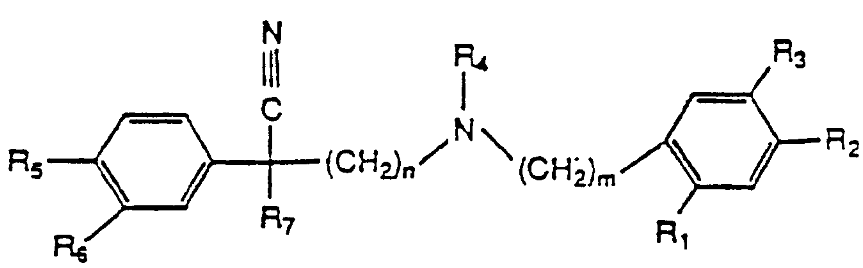

et des sels pharmaceutiquement acceptables dudit composé, formule dans laquelle :
$R_1$ est un groupe hydroxy ; un groupe alkyle, alcényle ou alcynyle substitué ou non substitué de 1 à 10 atomes de carbone, dans lequel le substituant est un groupe hydroxy ou céto ; un groupe alkylcarbonyle de 1 à 10 atomes de carbone ;
un groupe alcoxycarbonyle de 1 à 10 atomes de carbone; ou un groupe alkylamido de 1 à 10 atomes de carbone;

$R_2$, $R_3$, $R_5$ et $R_6$ désignent indépendamment un groupe hydroxy ; ou alcoxy de 1 à 10 atomes de carbone ; et
$R_4$ et $R_7$ représentent indépendamment un atome d'hydrogène ; un groupe alkyle, alcényle, ou alcynyle de 1 à 10 atomes de carbone ;
m est un nombre entier de 1 à 2 inclus ; et
n est un nombre entier de 1 à 5 inclus ;
pour la préparation d'un médicament pour l'immunosuppression chez un mammifère.

2. Utilisation d'un composé de formule générale :

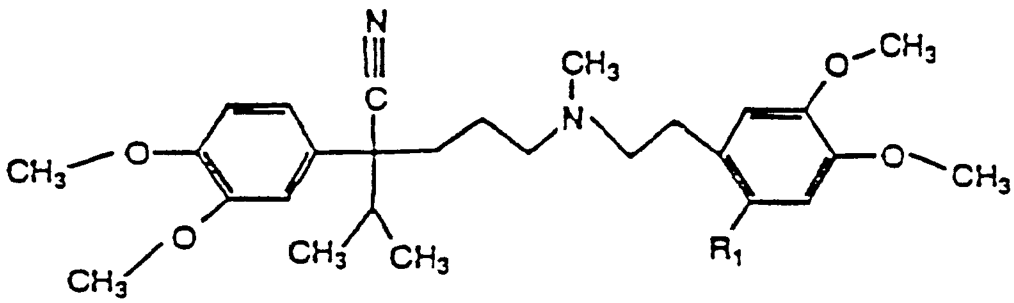

et des sels pharmaceutiquement acceptables dudit composé, formule dans laquelle
$R_1$ est un groupe hydroxy;
un groupe alkyle, alcényle ou alcynyle substitué ou non substitué de 1 à 10 atomes de carbone dans lequel ledit substituant est un groupe céto ou hydroxy ; un groupe alkylcarbonyle de 1 à 10 atomes de carbone ; un groupe alcoxycarbonyle de 1 à 10 atomes de carbone ; ou bien un groupe alkylamido de 1 à 10 atomes de carbone, pour la préparation d'un médicament pour l'immunosuppression chez un mammifère.

3. Utilisation selon la revendication 1, selon laquelle ledit composé a la formule structurale :

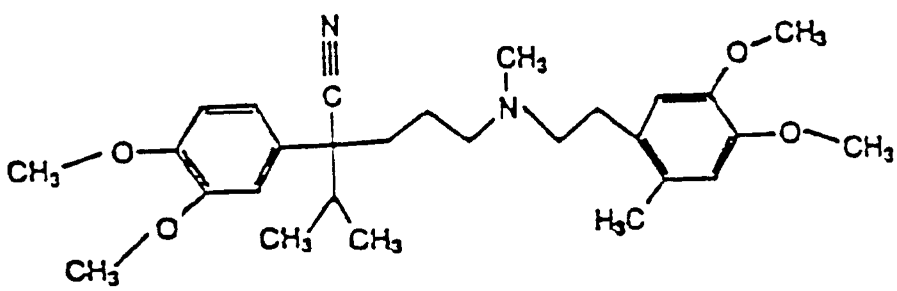

4. Utilisation selon la revendication 1, selon laquelle ledit composé a la formule structurale :

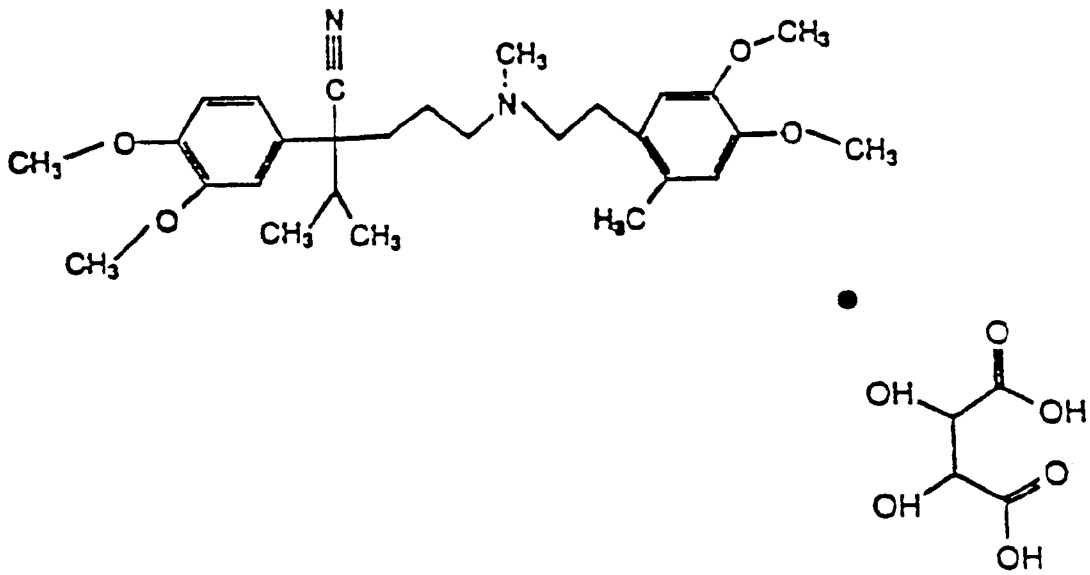

5. Utilisation selon la revendication 1, selon laquelle le composé a la formule structurale :

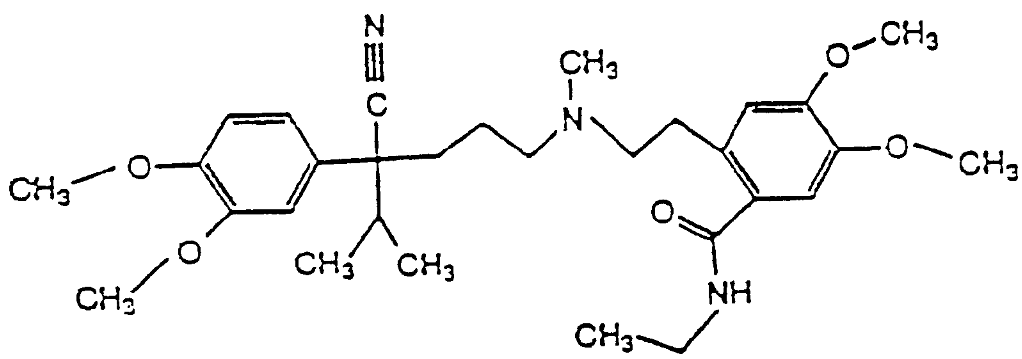

6. Utilisation selon la revendication 1, selon laquelle ledit composé a la formule structurale :

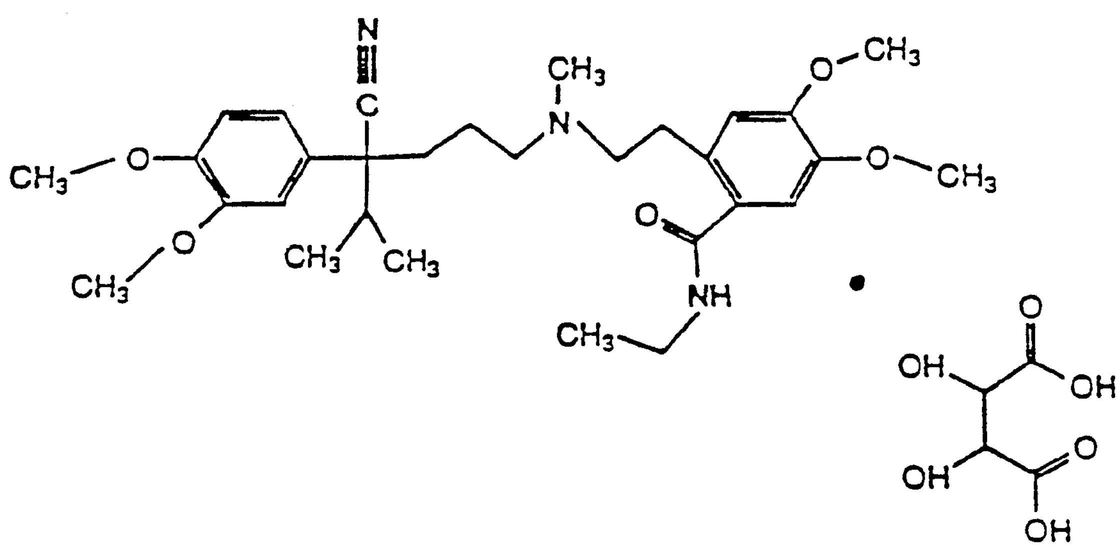

7. Utilisation selon la revendication 1, selon laquelle ledit composé a la formule structurale :

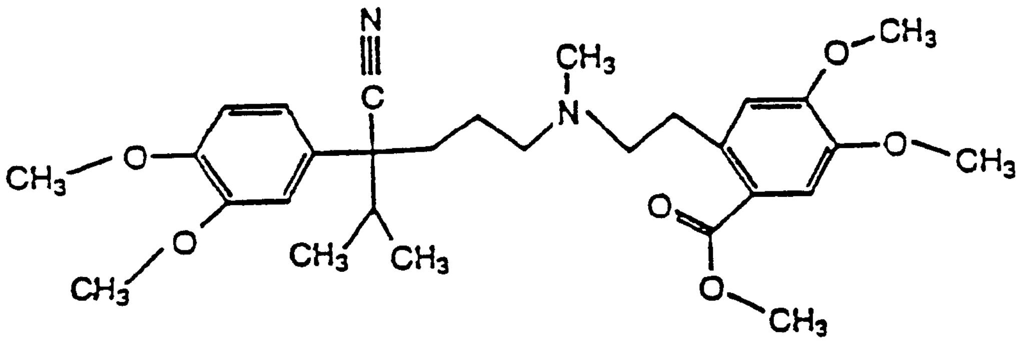

8. Utilisation selon la revendication 1, selon laquelle ledit composé a la formule structurale :

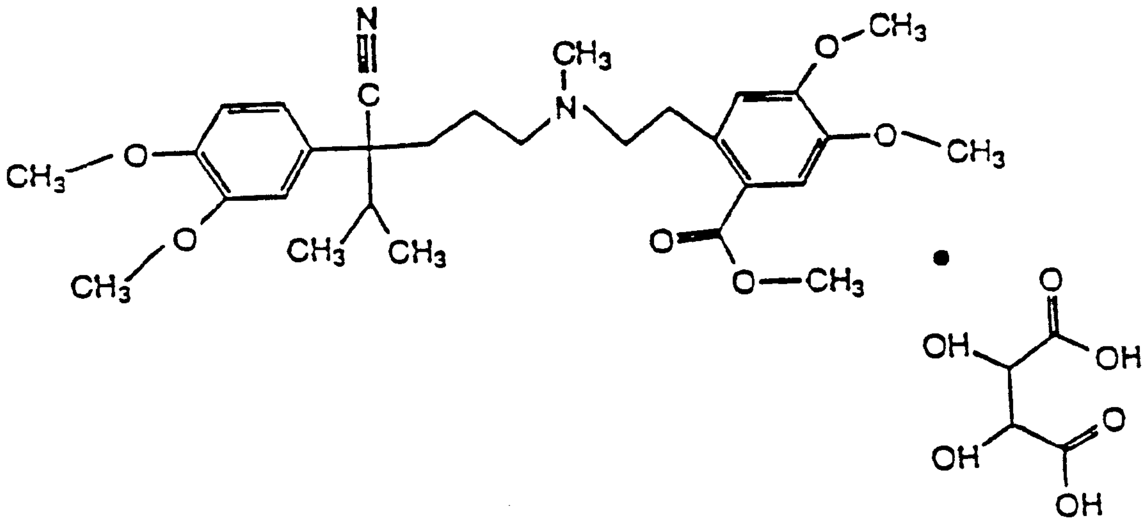

9. Utilisation selon la revendication 1, selon laquelle ledit composé a la formule structurale :

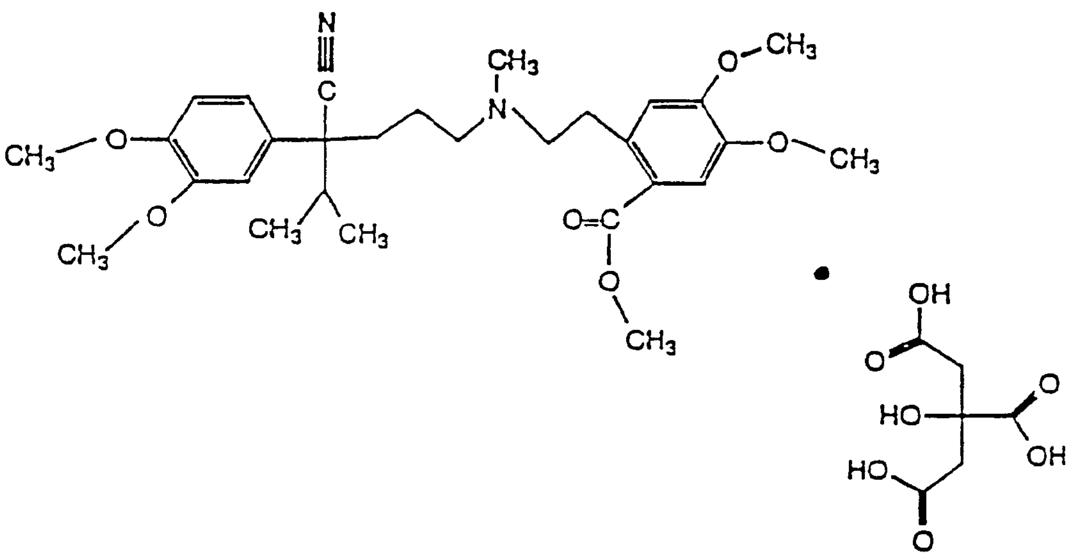

10. Utilisation selon la revendication 1, selon laquelle ledit composé a la formule structurale :

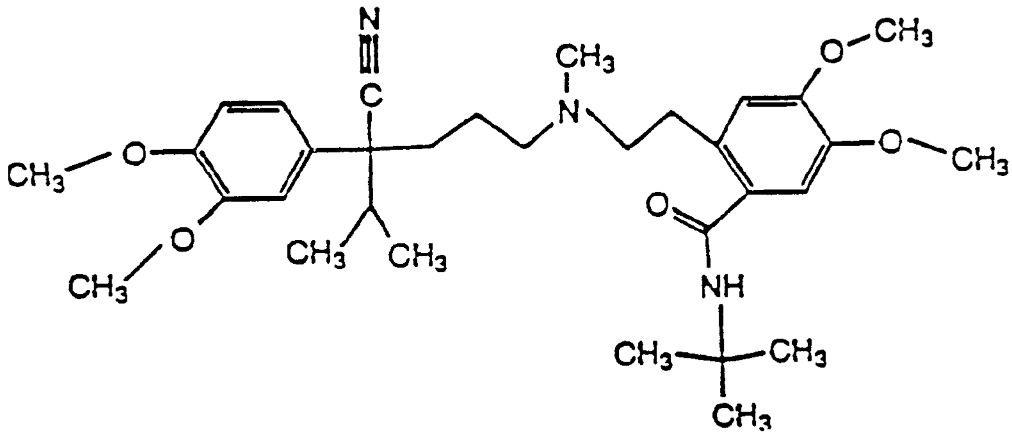

11. Utilisation selon la revendication 1, selon laquelle ledit composé a la formule structurale :

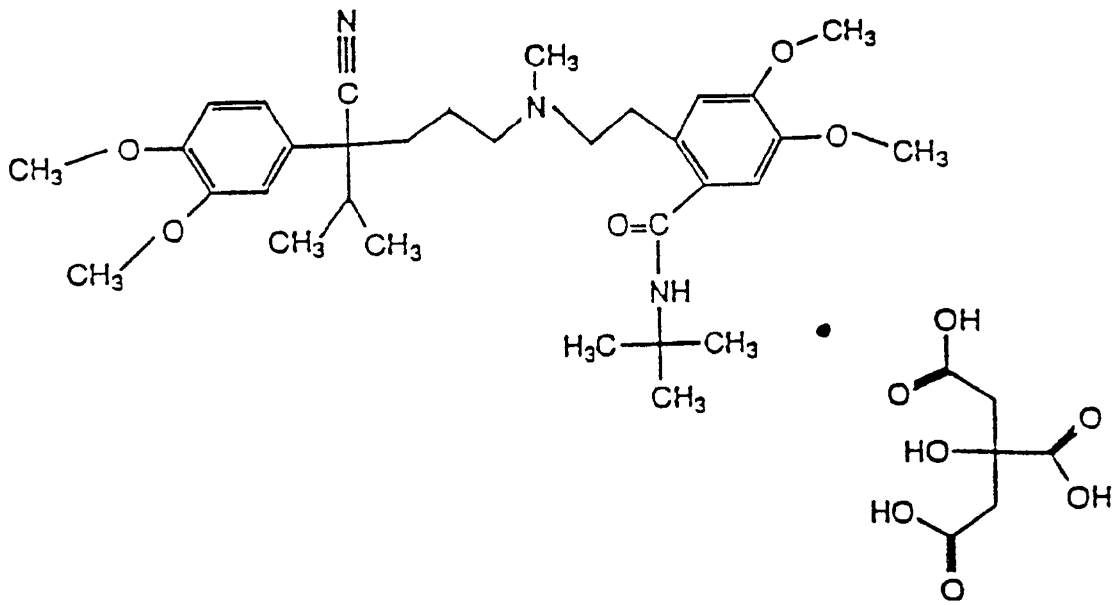

12. Utilisation selon la revendication 1, selon laquelle ledit composé a la formule structurale :

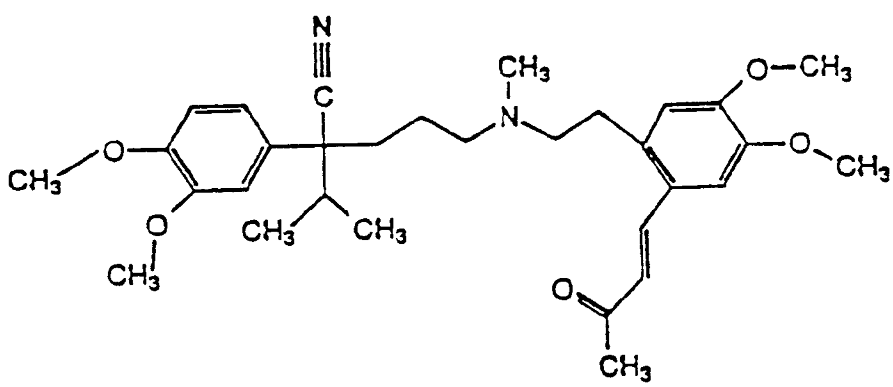

13. Utilisation d'un composé tel que défini dans la revendication 1 pour la préparation d'un médicament pour traiter l'hypertension chez un mammifère.

14. Utilisation d'un composé tel que défini dans la revendication 1 pour la préparation d'un médicament pour le traitement de l'ischémie rénale chez un mammifère.

15. Utilisation d'un composé tel que défini dans la revendication 1 pour la préparation d'un médicament pour le traitement de l'ischémie cérébrale chez un mammifère.